# EUROPÄISCHE PATENTANMELDUNG

(11) **EP 1 205 177 A2**
(43) Veröffentlichungstag der Anmeldung: **15.05.2002**
(21) Anmeldenummer: 01126788.7
(22) Anmeldetag: 09.11.2001
(51) Int. Cl.: A61K 7/42

(54) **Konjugat, dessen Herstellung und Verwendung**

(30) Priorität: 09.11.2000 DE 10055469
(71) Anmelder: MERCK PATENT GmbH, 64293 Darmstadt (DE)
(72) Erfinder: Pflücker, Frank, 64285 Darmstadt (DE); Anselmann, Ralf, 67305 Ramsen (DE); Kirschbaum, Michael, 64521 Gross-Gerau (DE); Buchholz, Herwig, 60599 Frankurt/ Main (DE); Driller, Hansjürgen, 64823 Gross-Umstadt (DE)
(74) Vertreter: Grünecker, Kinkeldey, Stockmair & Schwanhäusser Anwaltssozietät

(57) **Zusammenfassung**

Die Erfindung betrifft ein Konjugat, das zur Herstellung von dermatologischen und kosmetischen Zusammensetzungen verwendet werden kann. Die Erfindung betrifft ebenfalls Verfahren zur Herstellung eines solchen Konjugats sowie dessen Verwendung.

## Beschreibung

Die vorliegende Erfindung betrifft ein Konjugat, das zur Herstellung von dermatologischen und kosmetischen Zusammensetzungen verwendet werden kann. Die Erfindung betrifft ebenfalls Verfahren zur Herstellung eines solchen Konjugats sowie dessen Verwendung.

Dermatologische oder kosmetische Zusammensetzungen werden z.B. verwendet, um die Haut vor schädlichen äußeren Einflüssen, wie z.B. vor Sonnenstrahlung, zu schützen. Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie z.B. Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verhindern können. Die in den pharmazeutischen oder kosmetischen Zubereitungen enthaltenen UV-Filter bilden auf der Oberfläche der Haut einen Film bzw. eine Schicht aus. Bekannte UV-Filter bzw. Sonnenschutzmittel wirken in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfaßt UVB-Strahlen mit einer Wellenlänge zwischen 280 und 320 nm, die bei der Bildung eines Sonnenerythems eine entscheidende Rolle spielen, sowie UVA-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Auf die Haut aufgebrachte Lichtschutzformulierungen haben die Aufgabe, die hautschädigenden Strahlungsanteile zurückzuhalten. Als Lichtfilter kommen anorganische oder organische Materialien in Frage.

Lichtschutzformulierungen auf der Basis organischer Lichtfilter enthalten organische Lichtfilter, die in Wasser und/oder Öl löslich sind oder die weder in Wasser noch in Öl löslich sind.

Lichtschutzformulierungen mit unlöslichen partikulären organischen Lichtfiltern werden z.B. in der WO 97/3643 beschrieben. Die Gruppe der unlöslichen organischen Lichtfilter ist jedoch auf wenige Verbindungsklassen beschränkt.

Lichtschutzformulierungen mit organischen Lichtfiltern, die in Wasser und/oder in Öl löslich sind, werden in den Veröffentlichungen DE-A-197 46 654, DE-A-197 55 504, EP-A-709 080, EP-A-775 698, EP-A-893 119 und US-A-5 882 632 beschrieben.

Die DE-A-197 46 654 beschreibt beispielsweise die Verwendung von 4,4-Diarylbutadienderivaten als lösliche organische Lichtfilter in Lichtschutzformulierungen zum Schutz der Haut gegen UVA-Strahlung.

Die zuvor genannten Lichtschutzformulierungen müssen die organischen Lichtfilter in einer hohen Konzentration enthalten, um einen ausreichenden Lichtschutz zu gewährleisten. Der gravierendste Nachteil organischer löslicher Lichtfilter besteht jedoch darin, dass sie auf Grund ihrer Löslichkeit gegebenenfalls in die Haut eindringen und Hautschädigungen oder Allergien verursachen können.

Die JP-A-11-255 630 beschreibt eine Lichtschutzformulierung zum Schutz der Haut gegen UVA-Strahlung, die ein Dibenzoylmethanderivat enthält, das auf einem mit einem Silikonpolymer beschichteten anorganischen Träger aufgebracht ist. Die Herstellung dieser Lichtschutzformulierung ist jedoch auf Grund der Vielzahl an Arbeitsschritten umständlich und zeitaufwendig. Weiterhin sind Dibenzoylmethanderivate nicht photostabil.

Dermatologische und kosmetische Zusammensetzungen können weiterhin eine Vielzahl von Wirkstoffen enthalten, wie z.B. organische Substanzen mit antioxidativen und/oder radikalinhibierenden Eigenschaften sowie Repellentien.

Generell ist eine Heterogenisierung der in den dermatologischen und kosmetischen Zusammensetzungen enthaltenen Wirkstoffe erstrebenswert, da u.a. eine Penetration in die Haut und eine möglicherweise daraus resultierende Hautschädigung oder Allergie verhindert werden kann.

Aufgabe der vorliegenden Erfindung ist es, ein Konjugat auf der Basis organischer Wirkstoffe bereitzustellen, das nicht in die Haut eindringen bzw. penetrieren kann.

Diese Aufgabe wird erfindungsgemäß durch die Bereitstellung eines Konjugats gelöst, das ein anorganisches Pigment und einen Wirkstoff auf der Basis organischer Verbindungen umfasst, der kovalent über eine Spacer-Gruppe an das anorganische Pigment gebunden ist. Das erfindungsgemäße Konjugat ist dadurch gekennzeichnet, dass die Spacer-Gruppe ein Siliciumatom oder ein Aluminiumatom enthält.

Die Erfindung stellt ebenfalls eine dermatologische oder kosmetische Zusammensetzung bereit, umfassend mindestens ein Konjugat der zuvor genannten Art und mindestens einen kosmetisch, pharmazeutisch und/oder dermatologisch verträglichen Träger und/oder Hilfsstoff.

Im Rahmen der vorliegenden Erfindung wird unter dem Begriff "Konjugat" das Produkt verstanden, das durch molekulare, d.h. kovalente Verknüpfung des Wirkstoffes mit dem anorganischen Pigment erhalten wird. Der Begriff "Wirkstoff" umfasst z.B. lichtabsorbierende organische Verbindungen, Substanzen mit antioxidativen und/oder radikalinhibierenden Eigenschaften, Repellentien, Konservierungsmittel und Derivate dieser Wirkstoffe, die kovalent über eine Spacer-Gruppe an das anorganische Pigment gebunden werden können. Der Wirkstoff oder das Derivat davon umfasst bevorzugt eine nucleophile Gruppe. Es ist bevorzugt, dass der Wirkstoff als solcher, d.h. ohne an das anorganische Pigment gebunden zu sein, wasserlöslich und/oder öllöslich ist.

Das erfindungsgemäße Konjugat enthält ein anorganisches Pigment. Im Rahmen der vorliegenden Erfindung wird unter dem Begriff "Pigment" ein Farb- oder Füllstoff verstanden, der im Anwendungsmedium unlöslich ist. Das anorganische Pigment in dem erfindungsgemäßen Konjugat ist bevorzugt ein Metall- oder Halbmetalloxid.

Beispiele für anorganische Pigmente umfassen Oxide, Silikate, Phosphate, Carbonate, Sulfate und Nitride, wobei Oxide bevorzugt verwendet werden.

Bevorzugte anorganische Pigmente umfassen Magnesiumoxid, Aluminiumoxid, Siliciumdioxid, Zinkoxid, Ceroxid, Titandioxid, Zirconiumoxid, Manganoxid, Boroxid, rotes oder schwarzes Eisenoxid, Talk, Kaolin, natürliche und synthetische Glimmermaterialien, wie z.B. Muscovit, Phlogopit, Lepidolit, Biotit und Vermiculit, Magnesiumcarbonat, Calciumcarbonat, Aluminiumsilikat, Bariumsilikat, Calciumsilikat, Magnesiumsilikat, Strontiumsilikat, Bariumsulfat, Calciumsulfat, Calciumphosphat, Fluorapatit, Hydroxyapatit, Keramikpulver, Bornitrid, Eisentitanat, Zeolith und Gemische davon. Siliciumdioxid, Titandioxid, Glimmer, Talk und Gemische der zuvor genannten Pigmente (im folgenden als "Mischpigmente" bezeichnet), wie z.B. Siliciumdioxid/Titandioxid, werden besonders bevorzugt verwendet.

Handelsüblich erhältliche anorganische Mischpigmente, die erfindungsgemäß verwendet werden können, umfassen Gemische von Titandioxid/Glimmer (Mica), Titandioxid/Glimmer/ Zinnoxid, Titandloxid/Glimmer/Eisenoxide, Titandioxid/Glimmer/Siliciumdioxid, Titandioxid/Giimmer/Karmin, Glimmer/Eisenoxide/Aiuminiumoxid, Glimmer/Eisenoxide, Titandioxid/Glimmer/Zinkoxid, Titandioxid/Glimmer/Bariumsulfat, Glimmer/Siliciumdioxid und Titandioxid/ Eisenoxide/Siliciumdioxid. Diese Mischpigmente werden unter den Bezeichnungen Timiron®, Soloron®, Colorona®, Dichrona®, Microna®, Micronasphe® und Ronaspher® vertrieben.

Pigmente, die Licht streuen, wie z.B. Ronaspher® LDP, sowie Perlglanzpigmente können ebenfalls verwendet werden.

Die Silikate können eine ketten-, band- oder blattförmige Struktur besitzen. Silikate mit einer blattförmigen Struktur, wie z.B. Glimmer oder Talk, werden bevorzugt verwendet.

Die Form, in der die Verbindung des Metalls oder Halbmetalls vorliegt, ist nicht auf bestimmte Formen beschränkt.

Die Verbindung des Metalls oder Halbmetalls liegt bevorzugt in Form von sphärischen Teilchen vor. Geeignete Materialien auf der Basis von Siliciumdioxid umfassen handelsüblich erhältliche Produkte, die unter der Bezeichnung Monospher®, wie z.B. Monospher® 10 (Siliciumdioxid mit einer Partikelgröße von 10 nm), Monospher® 25 (Siliciumdioxid mit einer Partikelgröße von 25 nm), Monospher® 100 (Siliciumdioxid mit einer Partikelgröße von 100 nm) oder Monospher® 500 (Siliciumdioxid mit einer Partikelgröße von 500 nm), oder Ronaspher® (Siliciumdioxid mit einer Partikelgröße von 50 nm bis 3 µm) angeboten werden.

Die Herstellung von monodispersen kugelförmigen Oxidpartikeln ist bekannt. Entsprechend dem Verfahren, das in der EP-A-216 278 beschrieben ist, können monodisperse kugelförmige Oxidpartikel durch hydrolytische Polykondensation von Alkoxiden erhalten werden.

Andere bevorzugte Formen, in denen die Verbindungen der Metalle oder Halbmetalle vorliegen können, umfassen Nadeln und Flocken.

Die Wirkstoffe auf der Basis organischer Verbindungen, die erfindungsgemäß kovalent an das anorganische Pigment gebunden werden, umfassen z.B. lichtabsorbierende organische Verbindungen, Substanzen mit antioxidativen und/oder radikalinhibierenden Eigenschaften, Repellentien sowie Konservierungsmittel. Die erfindungsgemäß verwendeten Wirkstoffe sind jedoch nicht auf diese Wirkstoffe beschränkt.

Die lichtabsorbierenden organischen Verbindungen werden aus Verbindungen ausgewählt, die UV-Licht absorbieren. Dabei werden Verbindungen eingesetzt, die UV-Licht im UVB-Bereich, d.h. im Bereich von 280 bis 320 nm, und/oder im UVA-Bereich, d.h. im Bereich von 320 bis 400 nm, absorbieren.

Die UVB-Filter besitzen vorzugsweise ein Absorptionsmaximum im Bereich von 300 bis 320 nm und können aus bekannten Substanzen, die in der Fachliteratur beschrieben werden, ausgewählt werden. Beispiele umfassen Derivate von Aminobenzoesäure, Zimtsäure, Salicylsäure, Benzylidenkampfer, Phenylbenzimidazol, Diphenylacrylat, Triazin, Triazol und vinylgruppenhaltigen Amiden.

Beispiele für Aminobenzoesäurederivate umfassen 4-Aminobenzoesäure, 4-Aminobenzoesäure-2,3-dihydroxypropylester, 4-[Bis(2-hydroxypropyl)amino]benzoesäureethylester, 4-(Dimethylamino)benzoesäure-2-ethylhexylester (z.B. Eusolex® 6007) und ethoxylierten 4-Aminobenzoesäureethylester (z.B. Uvinul® P25).

Beispiele für Zimtsäurederivate umfassen Zimtsäureester, wie p-Methoxyzimtsäure-2-ethylhexylester (z.B. Eusolex® 2292), 4-Methoxyzimtsäureisopentylester, z.B. als Gemisch der Isomere (z.B. Neo Heliopan® E 1000), und 4-Methoxyzimtsäure-2-methylhexylester, sowie das Diethanolaminsalz von 4-Methoxy-Zimtsäure und Zimtsäurederivate, die in der US-A-5601811 und in der WO 97/851 beschrieben werden.

Zu den Salicylsäurederivaten zählen beispielsweise 2-Ethylhexylsalicylat (z.B. Eusolex® OS), 4-lsopropylbenzylsalicylat (z.B. Megasol®) und 3,3,5-Trimethylcyclohexylsalicylat (z.B. Eusolex® HMS).

Beispiele für Benzylidenkampferderivate umfassen 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)aniliniummethylsulfat (z.B. Mexoryl® SK) und α-(2-Oxoborn-3-yliden)toluol-4-sulfonsäure (z.B. Mexoryl® SL).

Als Beispiele für Phenylbenzimidazolderivate können 2-Phenylbenzimdazol-5-sulfonsäure und ihre Kalium-, Natrium- und Triethanolaminsalze genannt werden (z.B. Eusolex® 232).

Spezielle Beispiele für Diphenylacrylatderivate umfassen 2-Cyan-3,3'-diphenylacrylsäure-2-ethylhexylester und 2-Cyan-3,3'-diphenylacrylsäureethylester.

Beispiele für Triazolderivate umfassen Benzotriazole, wie z.B. 2-(2-Hydroxy-5-methylphenyl)benzotriazol, sowie die Triazole, die in der EP-A-893119 beschrieben werden.

Spezielle Beispiele für Triazine umfassen 2,4,6-Tri-{-4-[(2-ethylhexyl)oxycarbonyl]-phenylamino}-1,3,5-triazin sowie die Verbindungen, die in der EP-A-893119 beschrieben werden. Weitere Beispiele umfassen Trianilin-Triazinderivate, wie sie in der US-A-5 332 568, EP-A-570838, EP-A-517104, US-A-5 252 323, WO 93/17002 und der WO 97/03642 offenbart werden, Hydroxyphenyltriazinderivate, wie sie in der EP-A-775698 beschrieben werden, sowie Bis-Resorcinoldialkylaminotriazine, wie sie beispielsweise in der EP-A-780382 offenbart werden.

Bevorzugte Beispiele für vinylgruppenhaltigen Amidderivate umfassen jene, die in der EP-A-893119 beschrieben werden.

Als UVA-Filtersubstanzen kommen vorzugsweise Verbindungen in Betracht, die ein Absorptionsmaximum im Bereich von 330 bis 360 nm besitzen. Es können beliebige bekannte UVA-Filtersubstanzen, wie beispielsweise Derivate von Benzophenon, Dibenzoylmethan, Dirarylbutadien und Triazin, verwendet werden.

Spezielle Beispiele für Benzophenonderivate umfassen 2-Hydroxy-4-methoxybenzophenon (z.B. Eusolex® 4360), 2-Hydroxy-4-methoxybenzophenon-5-sulfonsäure und ihr Natriumsalz (z.B. Uvinul® MS-40), sowie 8-(2,2'-Dihydroxy-4-methoxybenzophenon).

Spezielle Beispiele für Benzoylmethanderivate und Dibenzoylmethanderivate umfassen 1-(4-tert-Butylphenyl)-3-(4-methoxyphenyl)propan-1,3-dion (z.B. Eusolex® 9020) und 4-Isopropyldibenzoylmethan (z.B. Eusolex® 8020).

Beispiele für Diarylbutadienderivate umfassen die 4,4-Diarylbutadiene, die in der DE-A-197 46 654 beschrieben werden, insbesondere 4,4-Diphenylbutadien.

Spezielle Beispiele für Triazine umfassen 2,4-Bis-{[4-(2-ethylhexyloxy)-2-hydroxy]-phenyl}-6-(4-methoxyphenyl)-1,3,5-triazin.

Weitere geeignete UV-Filter umfassen 2-Cyano-3,3-diphenylacrylsäure-2-ethylhexylester (z.B. Eusolex® OCR), 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX), 2,4,6-Trianilino-(p-carbo-2'-ethylhexyl-1'-oxi)-1,3,5-triazin ( z.B. Uvinul® T 150), 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®), 4,4'-[(6-[4-((1,1-Dimethyl-ethyl)-aminocarbonyl)phenylamino]-1,3,5-triazin-2,4-diyl)diimino]bis(benzoesäure-2-ethylhexylester) (z.B. Uvasorb® HEB), α-(Trimethylsilyl)-ω-[trimethylsilyl)oxy]poly[oxy-(dimethyl [und ca. 6% methyl[2-[p-[2,2-bis(ethoxycarbonyl]vinyl]phenoxy]-1-methylene-thyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1), 2,2'-Methylen-bis-(6-(2H-benzotriazol-2-yl)-4-(1,1,3,3-tetramethylbutyl)phenol) (CAS-Nr. 103 597-45-1), 2,2'-(1,4-Phenylen)bis-(1H-benzimidazol-4,6-disulfonsäure, Mononatriumsalz) (CAS-Nr. 180 898-37-7) und 2,4-bis-{[4-(2-Ethyl-hexyloxy)-2-hydroxyl]-phenyl}-6-(4-methoxy-phenyl)-1,3,5-triazin (CAS-Nr. 103 597-45-, 187 393-00-6).

Bevorzugte UV-Strahlung absorbierende organische Verbindungen sind 3-(4'-Methylbenzyliden)-dl-kampfer, 1-(4-tert-Butylphenyl)-3-(4-methoxyphenyl)propan-1,3-dion, 4-Isopropyl-dibenzoylmethan, 2-Hydroxy-4-methoxybenzophenon, Methoxyzimtsäureoctylester, Methoxyzimtsäureoctylester, 3,3,5-Trimethylcyclohexylsalicylat, 4-(Dimethylamino)-benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Photostabile UV-Strahlung absorbierende organische Verbindungen werden bevorzugt verwendet, wobei photostabile UVA-Filter und UVB-Filter besonders bevorzugt verwendet werden.

Geeignete Substanzen mit antioxidativen und/oder radikalinhibierenden Eigenschaften umfassen z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide wie z.B. D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester), Diaurylthiodipropionat, Distearylthiodipropionat, Thiodipropiosäure und deren Derivate (Ester, Ether, Peptide, Lipide, Nukleotide und Nukleoside) sowie Sulfoximinverbindungen (z.B. Buthioninsulfoximine, Homocysteinsulfoximin, Buthioninsulfone, Penta-, Hexa-, Heptathioninsulfoximin), Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Vitamin E und dessen Derivate, Stilbene und deren Derivate (z.B. Stilbenoxid, trans-Stilbenoxid) sowie BHT (2,6-Di-tert-Butyl-4-methyl-phenol).

Bevorzugte Antioxidantien umfassen Flavonoide, Coumaranone, Vitamine und BHT.

Als Flavanoide werden die Glycoside von Flavanonen, Flavonen, 3-Hydroxyflavonen (= Flavanolen), Auronen, Isoflavonen und Rotenoiden aufgefaßt (Römpp Chemie Lexikon, Band 9, 1993). Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavanone sind durch folgende Grundstruktur gekennzeichnet:

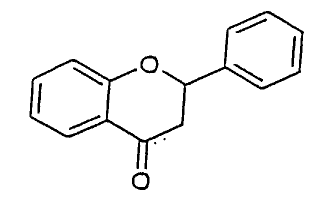

Die Flavone sind durch folgende Grundstruktur gekennzeichnet:

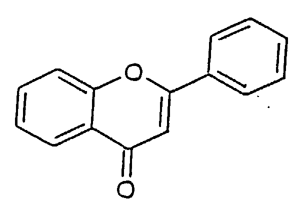

Die 3-Hydroxyflavone (Flavonole) sind durch folgende Grundstruktur gekennzeichnet:

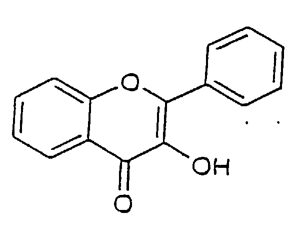

Die Isoflavone sind durch folgende Grundstruktur gekennzeichnet:

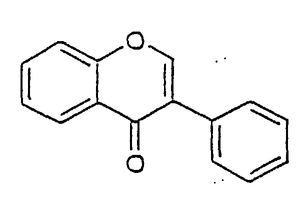

Die Aurone sind durch folgende Grundstruktur gekennzeichnet:

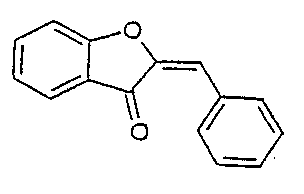

Die Coumaranone sind durch folgende Grundstruktur gekennzeichnet:

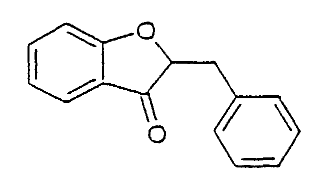

Vorzugsweise werden die Flavonoide und Coumaranone ausgewählt aus den Verbindungen der Formel (1): 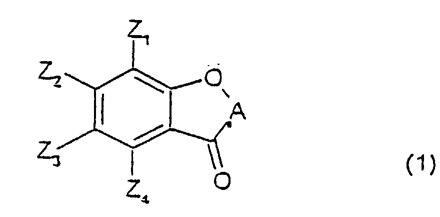 worin bedeuten:
- Z₁ bis Z₄: jeweils unabhängig voneinander H, OH, Alkoxy, Hydroxyalkoxy, Mono- oder Oligoglycosidreste, wobei die Alkoxy- und Hydroxyalkoxygruppen verzweigt und unverzweigt sein und 1 bis 18 Kohlenstoffatome aufweisen können und wobei an die Hydroxygruppen der genannten Reste auch Sulfat oder Phosphat gebunden sein kann,
- A: ausgewählt wird aus der Gruppe, bestehend aus den Teilformen (1A), (1 B) und (1C) 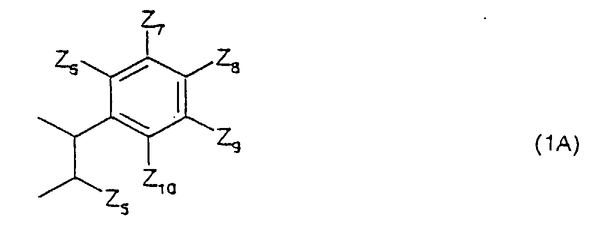 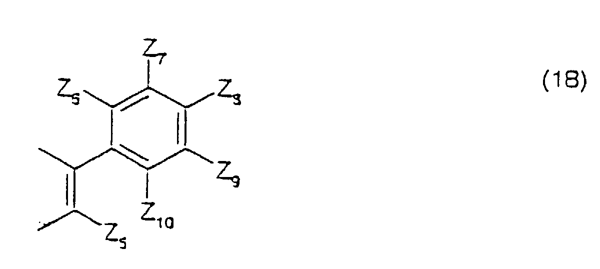 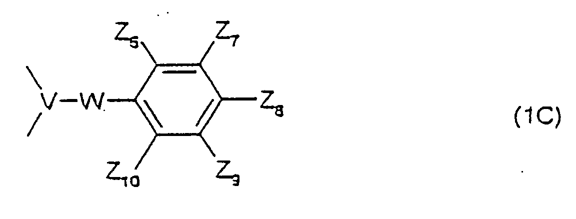
- Z₅: H, OH oder OR,
- R: einen Mono- oder Oligoglycosidrest,
- Z₆ bis Z₁₀: die Bedeutung der Reste Z₁ bis Z₄ besitzen, und

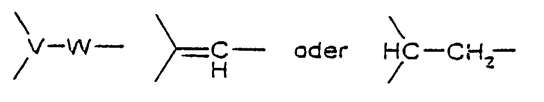

Die Alkoxygruppen sind vorzugsweise linear und besitzen 1 bis 12, vorzugsweise 1 bis 8 Kohlenstoffatome. Diese Gruppen entsprechen somit der Formel -O-(CH₂)ₘ-H, wobei m 1,2,3,4,5,6,7 oder 8 und insbesondere 1 bis 5 bedeutet.

Die Hydroxyalkoxygruppen sind vorzugsweise linear und besitzen 2 bis 12, vorzugsweise 2 bis 8 Kohlenstoffatome. Diese Gruppen entsprechen somit der Formel -O-(CH₂)ₙ-OH, wobei n 2,3,4,5,6,7 oder 8, insbesondere 2 bis 5 und besonders bevorzugt 2 bedeutet.

Die Mono- und Oligoglycosidreste sind vorzugsweise aus 1 bis 3 Glycosideinheiten aufgebaut. Vorzugsweise werden diese Einheiten ausgewählt aus der Gruppe der Hexosylreste, insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl, sind gegebenenfalls vorteilhaft zu verwenden. Es kann auch erfindungsgemäß vorteilhaft sein, Pentosylreste zu verwenden.

In einer bevorzugten Ausführungsform besitzen
- Z₁ und Z₃: die Bedeutung H,
- Z₂ und Z₄: eine andere Bedeutung als H, insbesondere bedeuten sie OH, Methoxy, Ethoxy oder 2-Hydroxyethoxy,
- Z₅: die Bedeutung H, OH oder einen Glycosidrest, der aus 1 bis 3, vorzugsweise aus 1 oder 2, Glycosideinheiten aufgebaut ist.
- Z₆, Z₉ und Z₁₀: die Bedeutung H, und
- Z₇ und Z₈: eine andere Bedeutung als H, insbesondere bedeuten sie OH, Methoxy, Ethoxy oder 2-Hydroxyethoxy.

Besonders bevorzugte Verbindungen werden durch die folgende allgemeine Formel dargestellt: 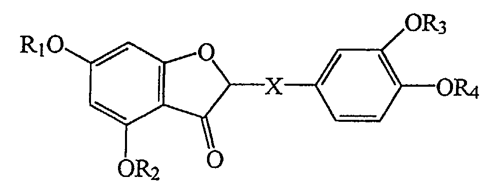 wobei - X - steht für eine Einfachbindung, - CH₂- oder = CH-, und
R¹, R², R³ und R⁴ gleich oder verschieden sein können, und unabhängig voneinander stehen für
H,
   geradkettige oder verzweigte C₁- bis C₁₂-Alkylgruppen und/oder
   - Alkylcarbonylgruppen
      geradkettige oder verzweigte C₃- bis C₁₂-Alkenylgruppen und/oder
   - Alkenylcarbonylgruppen
   geradkettige oder verzweigte C₁- bis C₁₂-Hydroxyalkyl- und/oder - Hydroxyalkylcarbonylgruppen, wobei die Hydroxygruppe an ein primäres oder sekundäres Kohlenstoffatom der Kette gebunden sein kann und weiter die Alkylkette auch durch Sauerstoff unterbrochen sein kann,

C₃- bis C₁₀-Cycloalkyl- und/oder -Cycloalkylcarbonylgruppen und
C₃- bis C₁₂-Cycloalkenyl- und/oder -Cycloalkenylcarbonylgruppen, wobei die Ringe auch durch -(CH₂)ₙ-Gruppen mit n = 1 bis 3 überbrückt sein können,
Arylgruppen und/oder Arylcarbonylgruppen
Heteroarylgruppen und/oder Heteroarylcarbonylgruppen
wobei diese Gruppen durch Alkyl-, Hydroxy-, Alkoxy-, Amino-, Mono- und Dialkylamino-, Sulfonsäure-, Carboxyl- und/oder Halogengruppen substituiert sein können,

Mono- oder Oligoglycosidreste, 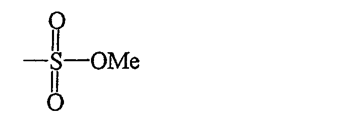 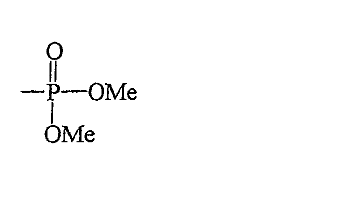 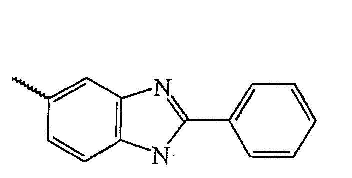 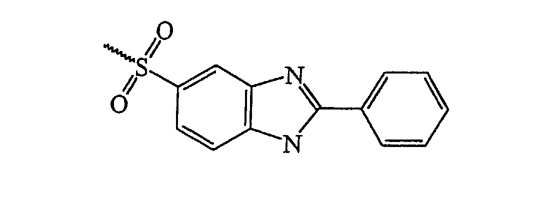 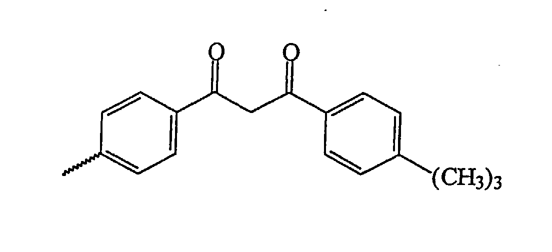 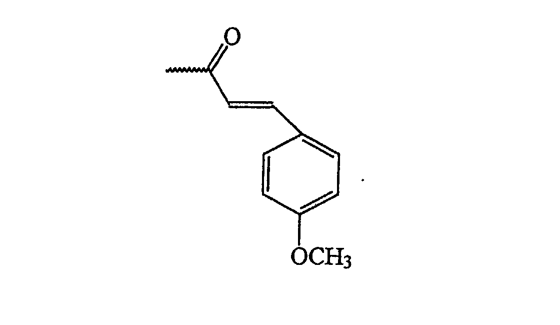 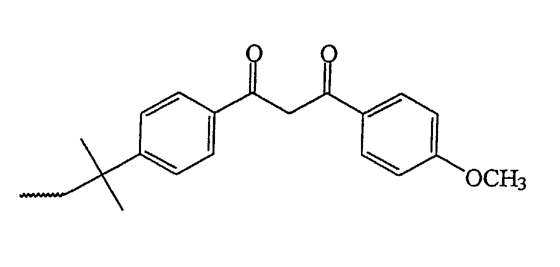 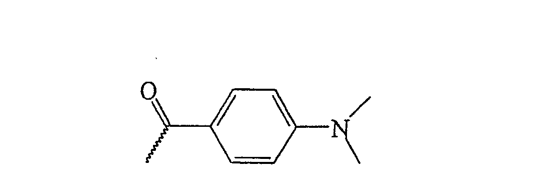 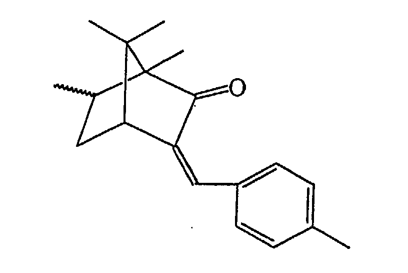 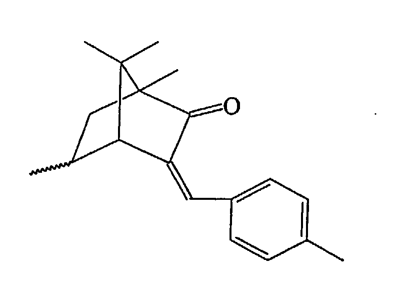 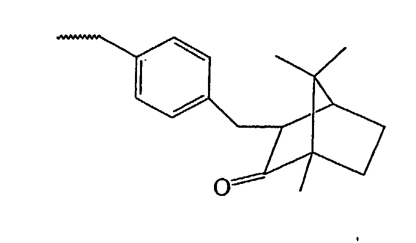 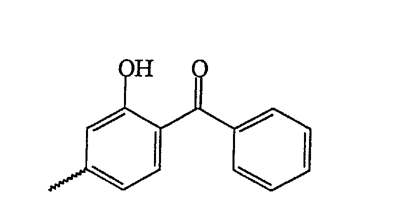 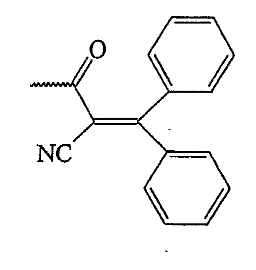 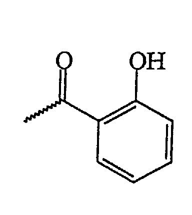 wobei R⁵ steht für tert.-Butyl oder Isopropyl und
Me steht für ein Proton oder ein Alkalimetallion, insbesondere ein Kaliumion.

Die Reste können also als Ether oder als Ester an den Grundkörper gebunden sein.

In einer weiteren bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin) sowie Trishydroxyethylluteolin (Troxeluteolin).

Unter den Flavonoiden sind insbesondere Rutin und Troxerutin bevorzugt. Besonders bevorzugt ist Troxerutin.

Unter den Coumaranonen ist 4,6,3',4'-Tetrahydroxybenzylcoumaranon-3 bevorzugt.

Geeignete Repellentien umfassen Amide und deren Derivate, insbesondere N,N-Diethyl-3-methyl-benzamid, 3-[N-n-Butyl-N-acetyl]-aminopropionsäureethylester (IR3535®) und N,N-Caprylsäurediethylamid (IR790®).

Geeignete Konservierungsmittel umfassen Benzalkoniumchlorid, Benzoesäure und deren Salze (wie z.B. Natriumbenzoat), Methylparaben, Ethylparaben, Propylparaben, Sorbinsäure und deren Salze (wie z.B. Kaliumsorbat), Cetylpyridiniumchlorid, Cetrimoniumchlorid sowie Salicylsäure und deren Salze (wie z.B. Natriumsalicylat).

Der erfindungsgemäß verwendete Wirkstoff kann ebenfalls eine entzündungshemmende Substanz sein.

Der erfindungsgemäß verwendete Wirkstoff ist bevorzugt öl- und/oder wasserlöslich und wird durch die kovalente Bindung an das anorganische Pigment in einen Zustand überführt, in dem er nicht mehr in die Haut eindringen bzw. penetrieren kann.

Bevorzugte erfindungsgemäße Konjugate sind durch die folgende allgemeine Formel dargestellt: 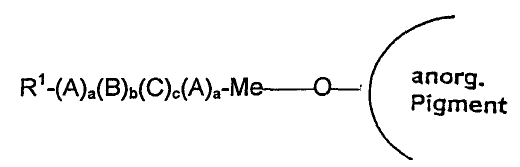 worin bedeuten:
- R¹: die kovalent gebundene Wirkstoffgruppe,
- A: O, S oder NH,
- B: eine geradkettige oder verzweigte Alkylengruppe mit bis zu 20, bevorzugt mit 1 bis 12, besonders bevorzugt mit 3 bis 12 Kohlenstoffatomen,
- C: eine geradkettige oder verzweigte Alkylenoxygruppe mit bis zu 20, bevorzugt mit 1 bis 12, besonders bevorzugt mit 3 bis 12 Kohlenstoffatomen, wobei das Sauerstoffatom der Alkylenoxygruppe an die Gruppe B gebunden ist,
- Me: ein Siliciumatom oder ein Aluminiumatom,
- a: 0 oder 1,
- b: 0 oder 1, und
- c: 0 oder 1.

Das Silicium- bzw. Aluminiumatom kann eine oder mehrere kovalente Bindungen zum anorganischen Pigment und/oder zur Wirkstoffgruppe ausbilden.

Bevorzugte Beispiele für R¹ umfassen neben den zuvor aufgeführten Substanzen: 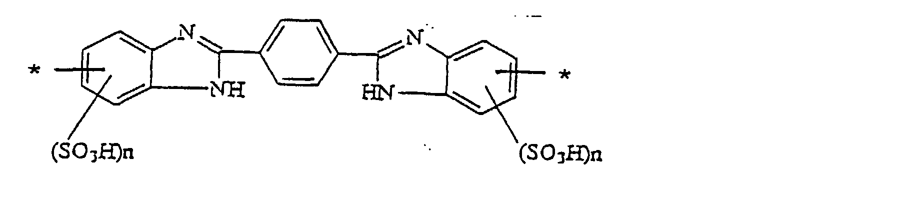 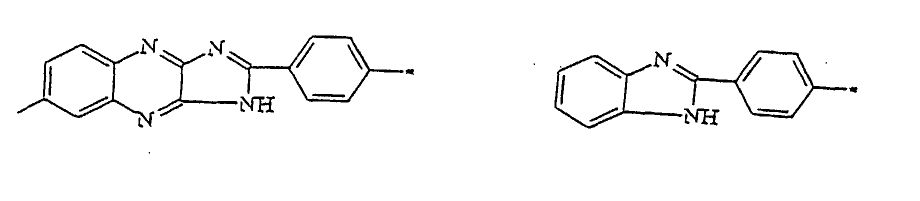 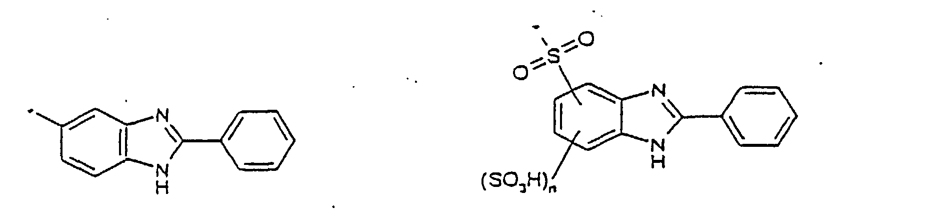 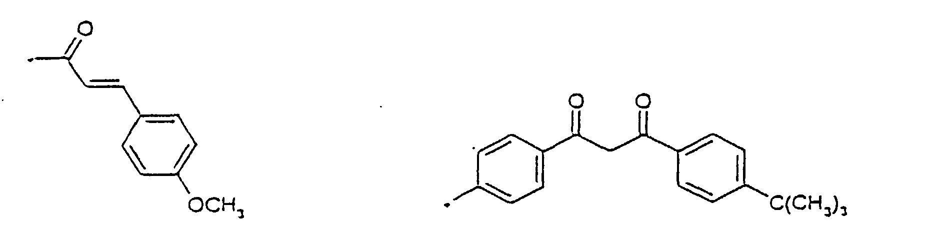 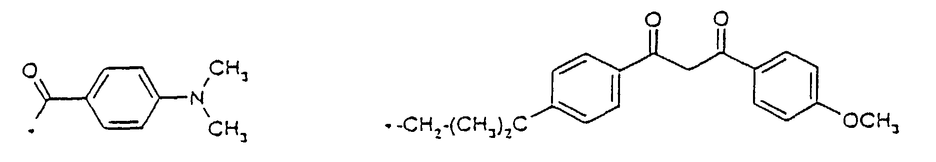 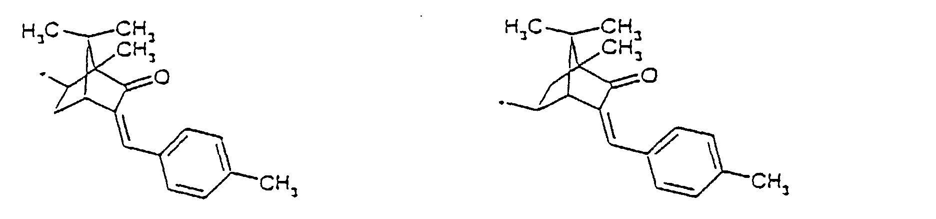 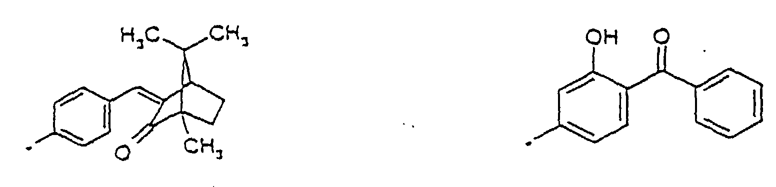 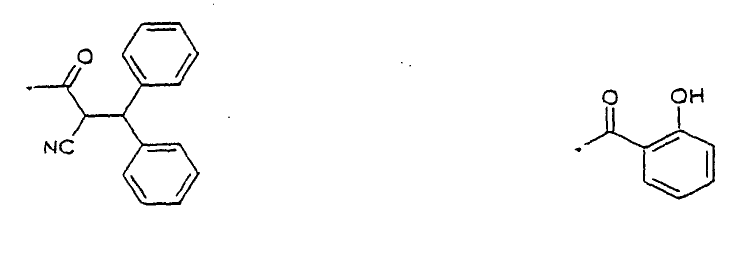 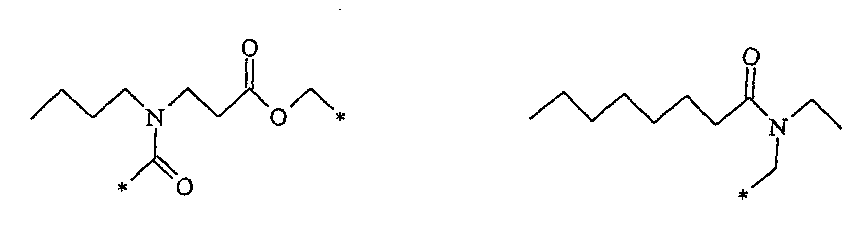 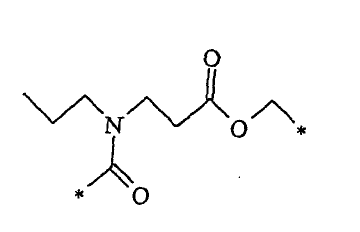 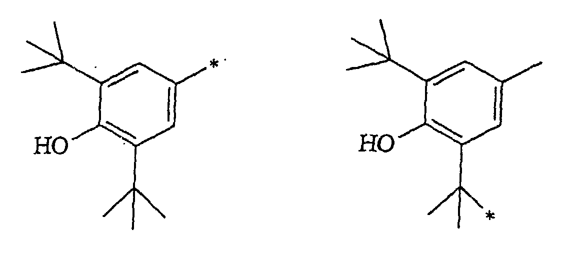 worin n 0, 1 oder 2 bedeutet, und * ist die Bindung zur Spacer-Gruppe.

Im folgenden wird die Herstellung des erfindungsgemäßen Konjugats beschrieben.

Zur Herstellung des erfindungsgemäßen Konjugats wird der Wirkstoff über eine Spacer-Gruppe (Verbindungsgruppe) kovalent an das anorganische Pigment gebunden. Im Rahmen der vorliegenden Erfindung wird unter dem Begriff "Spacer-Gruppe" eine monomere oder oligomere Verbindungsgruppe, jedoch keine polymere Gruppe, verstanden.

Die Spacer-Gruppe ist bevorzugt eine Verbindungsgruppe, die durch die folgende Formel dargestellt ist:

*-(A)ₐ(B)_{b}(C)_{c}(A)ₐ-Me-O-⁺

worin bedeuten:
- A: O, S oder NH,
- B: eine geradkettige oder verzweigte Alkylengruppe mit bis zu 20, bevorzugt mit 1 bis 12, besonders bevorzugt mit 3 bis 12 Kohlenstoffatomen,
- C: eine geradkettige oder verzweigte Alkylenoxygruppe mit bis zu 20, bevorzugt mit 1 bis 12, besonders bevorzugt mit 3 bis 12 Kohlenstoffatomen, wobei das Sauerstoffatom der Alkylenoxygruppe an die Gruppe B gebunden ist,
- Me: ein Siliciumatom oder ein Aluminiumatom,
- a: 0 oder 1,
- b: 0 oder 1,
- c: 0 oder 1,
- *: die Bindung zur Wirkstoffgruppe und
- +: die Bindung zum anorganischen Pigment.

Das erfindungsgemäße Konjugat kann hergestellt werden, indem zunächst eine Verbindung mit einem Silicium- oder Aluminiumatom, die mindestens zwei endständige reaktive Gruppen enthält, mit dem Wirkstoff auf der Basis organischer Verbindungen umgesetzt wird, um beide Verbindungen über eine der beiden reaktiven Gruppen kovalent aneinander zu binden. Das erhaltene Zwischenprodukt wird dann mit dem anorganischen Pigment umgesetzt, um das Zwischenprodukt kovalent über die andere reaktive Gruppe an das anorganische Pigment zu binden. 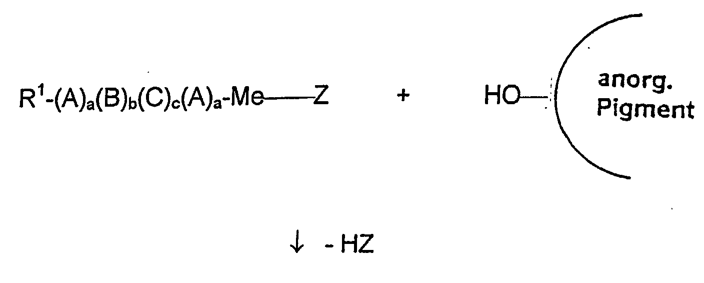 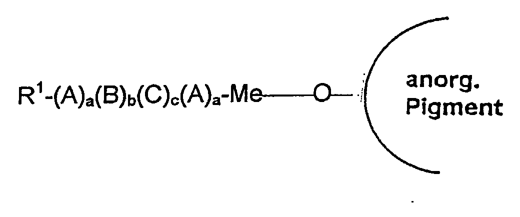 worin Z eine Gruppe, die abgespalten werden kann, wie z.B. ein Halogenatom (bevorzugt Cl) oder eine Alkoxygruppe, bedeutet.

Beispiele für die Wirkstoffgruppe R¹ umfassen die zuvor genannten Gruppen.

Beispielsweise kann 1-(4-tert-Butylphenyl)-3-(4-allyloxyphenyl)propan-1,3-dion (ein Eusolex® 9020-Analogon) mit Triethoxysilan wie folgt umgesetzt werden:

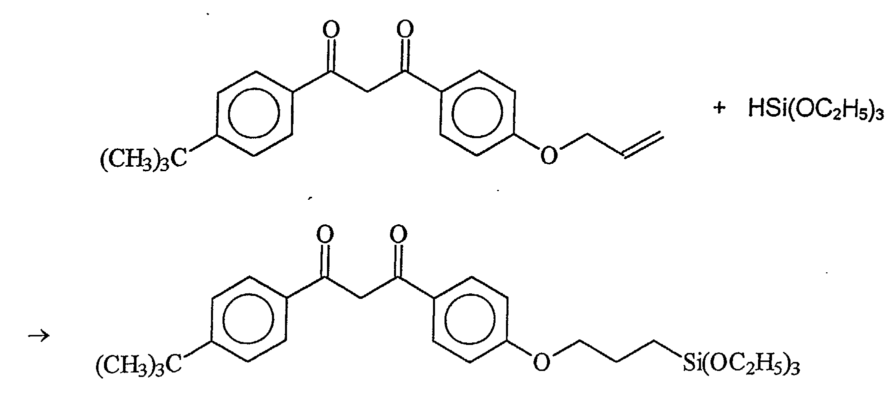

Das silylierte Zwischenprodukt wird dann kovalent an das anorganische Pigment, wie z.B. Siliciumdioxid, gebunden. Dabei reagieren die Ethoxygruppen des Zwischenproduktes mit den Hydroxygruppen, die an der Oberfläche des Siliciumdioxids gebunden sind, unter Abspaltung von Ethanol.

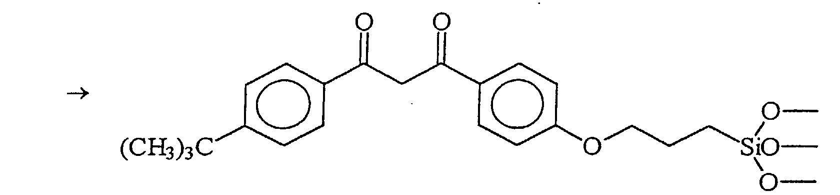

Diese Variante hat den Vorteil, dass die Zwischenprodukte gereinigt werden können und somit eine höhere chemische Reinheit des Endproduktes erreicht werden kann.

Alternativ kann das erfindungsgemäße Konjugat hergestellt werden, indem zunächst eine Verbindung mit einem Silicium- oder Aluminiumatom, die mindestens zwei endständige reaktive Gruppen enthält, mit einem anorganischen Pigment umgesetzt wird, um die Verbindung und das Pigment über eine der beiden reaktiven Gruppen kovalent aneinander zu binden. Das erhaltene Zwischenprodukt wird dann mit dem Wirkstoff auf der Basis organischen Substanzen umgesetzt, um das Zwischenprodukt kovalent über die andere reaktive Gruppe an den Wirkstoff zu binden. Diese Variante hat präparative Vorteile (Einsatz von hohen Überschüssen an Reagenzien, einfache Isolierung usw.).

Es ist ebenfalls möglich, das anorganische Pigment zunächst mit geeigneten, handelsüblich erhältlichen Siliciumverbindungen zu belegen. Auf diese Weise ist eine Vielzahl verschieden funktionalisierter Partikel zugänglich. Anschließend lassen sich die einzelnen Funktionalitäten zum Aufbau der Konjugate nutzen.

Auf diese Weise können z.B. die folgenden Konjugate erhalten werden:
(in den Formeln stellt die Gruppe R eine Alkylgruppe dar und die Gruppe X ist eine Gruppe, die abgespalten werden kann)

### Synthese eines Eusolex® 4360-Analogon/SiO₂-Konjugats

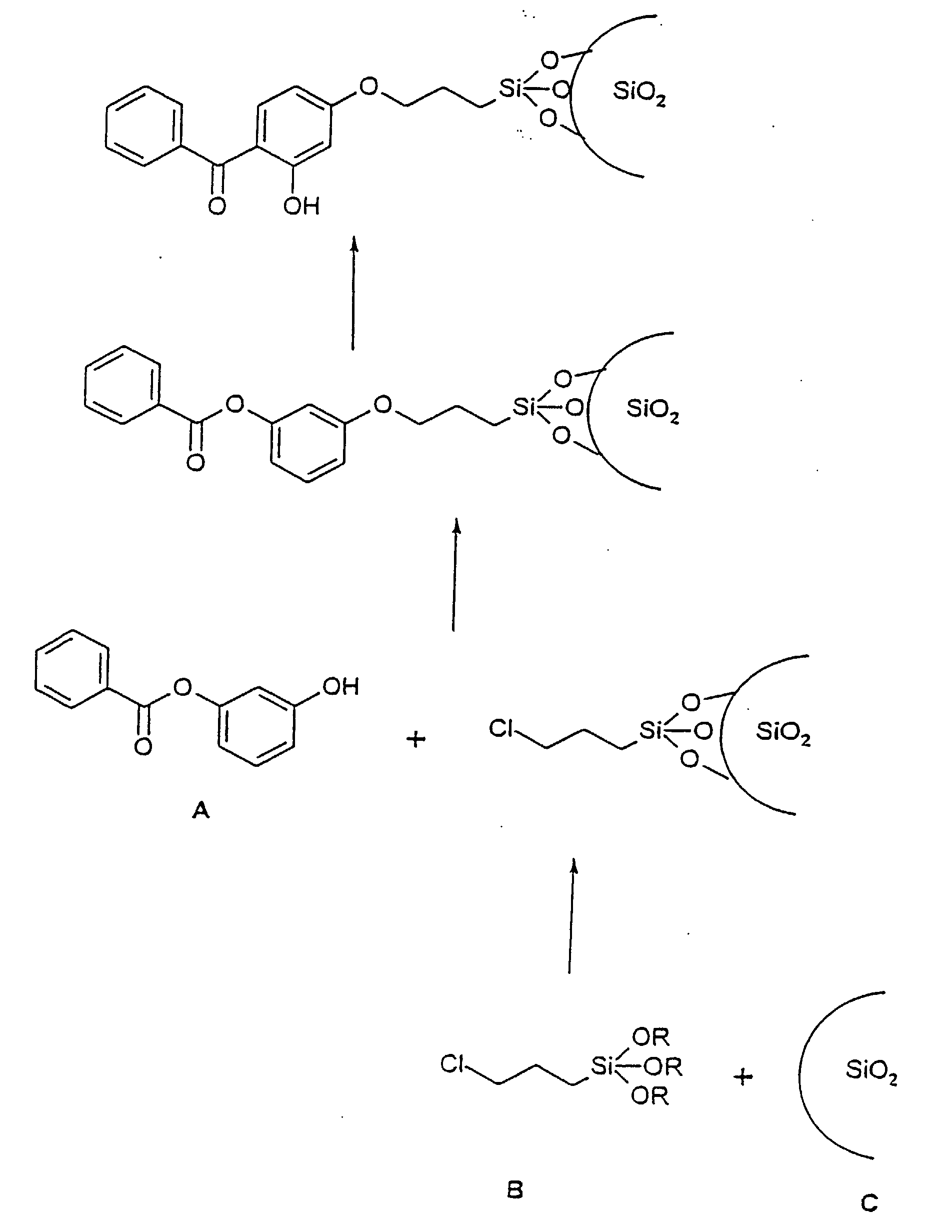

### Synthese eines Eusolex® 232-Analogon/SiO₂-Konjugats

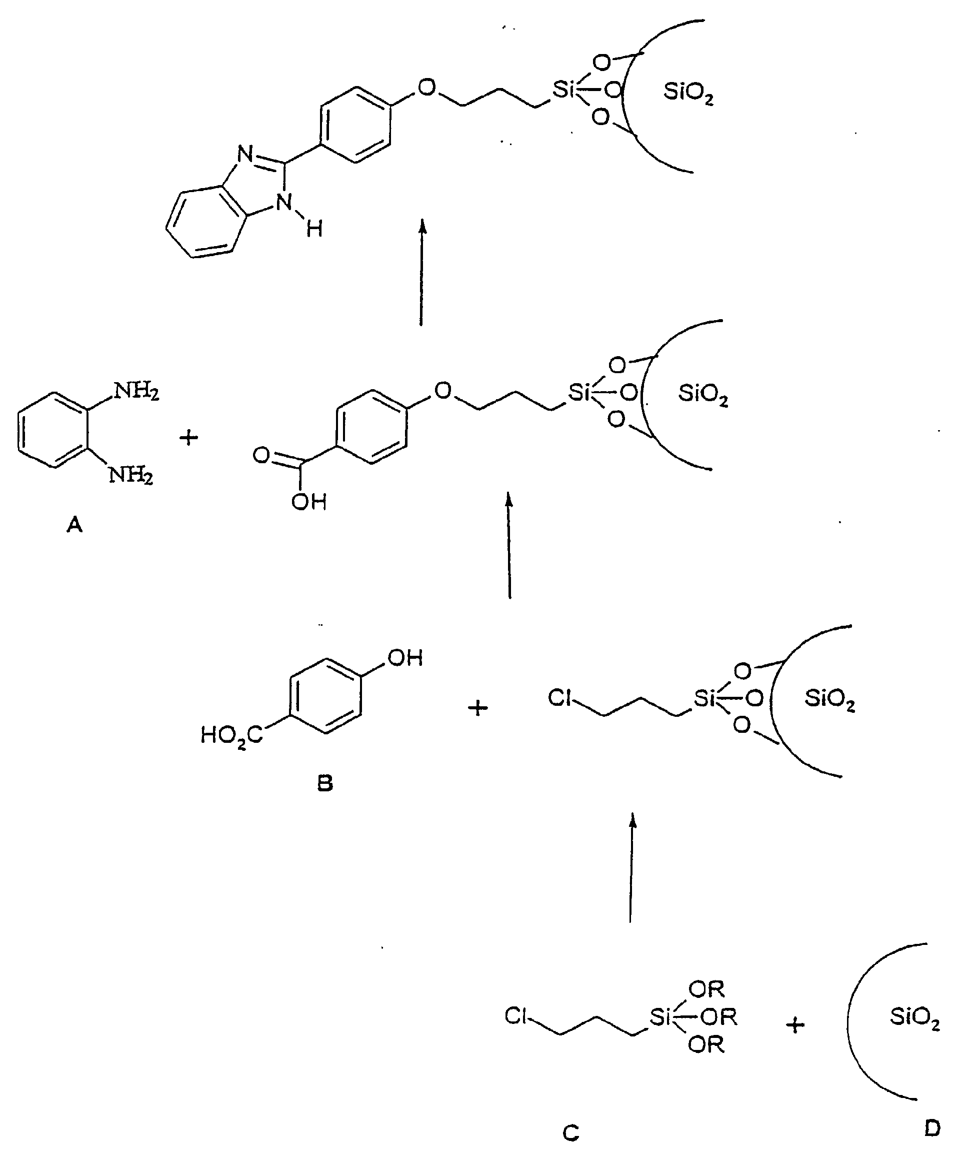

### Synthese eines Eusolex® 2292-Analogon/SiO₂-Konjugats

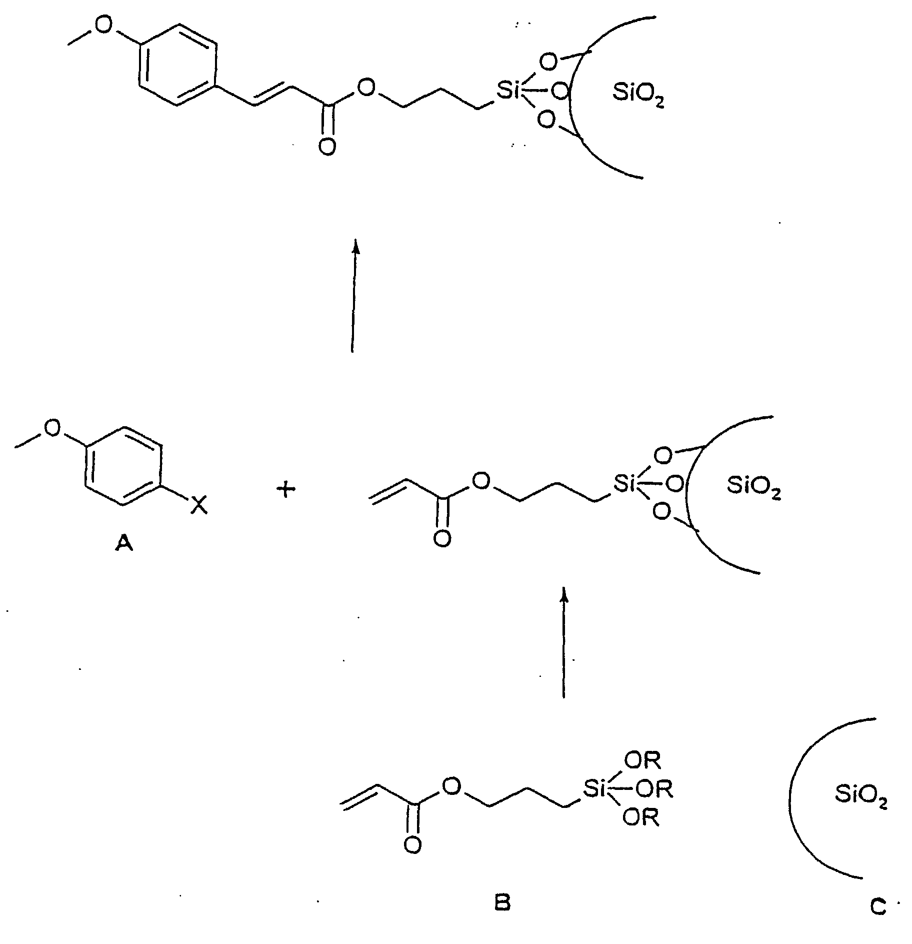

Altemativ kann im ersten Schritt die Kupplung von A mit B erfolgen. Im zweiten Schritt erfolgt dann die Belegung des anorganischen Pigments.

Die Synthese des erfindungsgemäßen Konjugats erfolgt in einem inerten Lösungsmittel, das in Abhängigkeit von den eingesetzten Ausgangsmaterialien ausgewählt wird. Wenn z.B. ein Alkoxysilan eingesetzt wird, wird bevorzugt der entsprechende Alkohol als Lösungsmittel verwendet.

Die Anzahl der reaktiven Gruppen, wie Hydroxygruppen, an der Oberfläche des anorganischen Pigments kann mit dem Verfahren, das in der DE-A-198 02 753 beschrieben ist, erhöht werden.

Anorganische Trägermaterialien weisen je nach chemischem Aufbau eine mehr oder weniger große Zahl an reaktiven OH-Gruppen an der Oberfläche auf, die eine chemische Bindung eingehen können. Diese Zahl beträgt z.B. für vollständig hydroxylierte SiO₂-Monosphären ca. 4,4 bis 8,5 pro nm² (H. P. Boehm, Angew. Chem. 78, 617 (1966)). Bestätigt wurden diese Werte durch J. Kratochvila et al., Journal of Non-Crystalline Solids 143, 14-20 (1992). Bei einem Bindungsabstand von ca. 0,16 nm für die Si-O-Bindung und einem Winkel von 150° für den Si-O-Si-Winkel befinden sich an der Oberfläche der SiO₂-Monosphären ca. 13 Si-Atome pro nm². Das bedeutet, dass in der oberflächigen Monoschicht maximal 13 Si-OH-Gruppen auftreten bei zusätzlicher 3-facher Valenzbindung des Si über die oxidischen Sauerstoffbrücken. In der Regel sind bei SiO₂-Monosphären, die bei Raumtemperatur getrocknet wurden, jedoch nur 4 Si-OH-Gruppen zu erwarten (vergl. Boehm und Kratochvila).

Um auf den SiO₂-Monosphären pro Gewichtseinheit eine möglichst große Anzahl aktiver Si-OH-Gruppen zu erhalten, können die Oberflächen der SiO₂-Monosphären in Form von Poren, Klüften und/oder durch möglichst kleine Partikeldurchmesser vergrößert werden. Eine Erhöhung der Anzahl der Si-OH-Gruppen durch Sättigung mit Wasser oder Wasserdampf führt zu keiner befriedigenden Lösung, denn das zusätzlich an der Oberfläche adsorbierte Wasser bewirkt bei den meisten chemisorptiven Belegungen der Oberfläche mit Liganden deren Hydrolyse. Starkes Trocknen wiederum führt gleichzeitig zu einer Abnahme der Anzahl der Si-OH-Gruppen auf bis unter 2 pro nm².

Entsprechend dem Verfahren, das in der DE-A-198 02 753 beschrieben wird, wird z.B. ein Oxid oder Silikat in einem inerten aprotischen Lösungsmittel, wie z.B. THF, DMF, Cyclohexan oder Toluol, mit einem stark basischen Reagenz, wie z.B. einem Alkali- oder Erdalkalihydroxid, einem Hydrid oder einem Alkoholat, behandelt, um die oberflächlichen Oxidbindungen des Oxids oder Silikats zu spalten, und das behandelte Oxid oder Silikat wird dann mit einer anorganischen oder organischen Säure behandelt, um zusätzliche Hydroxygruppen zu erzeugen.

Wenn die erfindungsgemäß verwendeten anorganischen Pigmente entsprechend diesem Verfahren behandelt werden, kann die Bindungsdichte der Wirkstoffe an der Oberfläche des anorganischen Pigments erhöht werden.

Die Partikelgröße des erfindungsgemäßen Konjugats ist nicht auf eine bestimmte Partikelgröße beschränkt; sie liegt jedoch gewöhnlich im Bereich von 1 nm bis 250 µm, bevorzugt im Bereich von 1 nm bis 1 µm und besonders bevorzugt im Bereich von 5 nm bis 100 nm, um eine optimale Verteilung auf der Haut zu gewährleisten.

Das erfindungsgemäße Konjugat kann nach dessen Herstellung getrocknet und dann in die dermatologische oder kosmetische Zusammensetzung eingebracht werden, oder es kann in Form einer Dispersion, z.B. dispergiert in einem kosmetischen Öl oder flüssigen Lichtfilter, in die dermatologische oder kosmetische Zusammensetzung eingebracht werden.

Verwendbare Ölkomponenten umfassen natürliche und synthetische Substanzen, wie z.B. Paraffinöl, Glycerylstearat, Isopropylmyristat, Diisorpopyladipat, 2-Ethylhexansäureacetylstearylester, hydriertes Polyisobuten, Vaseline, Caprylsäure/Caprinsäure-Triglyceride, mikrokristallines Wachs, Lanolin, Mineralöle, Mineralwachse, Ester von Fettsäuren mit Alkoholen, wie z.B. Isopropanol, Propylenglykol oder Glycerin, Alkylbenzoate, Silikonöle, wie z.B. Dimethylpolysiloxane, Diethylpolysiloxane oder Diphenylpolysiloxane, Stearinsäure, Lichtfilter, die in flüssiger Form vorliegen, sowie Vitamin K1. Bevorzugte Beispiele umfassen Ethylbutylacetylaminopropionat (IR 3535™), Butylenglycoldicaprylat/dicaprinat (Miglyol 8810), Propylenglycoldicaprylat/dicaprinat), C12-15 Alkylbenzoate, Isopropylmyristat, Caprylsäure/-Caprinsäure-Triglyceride, Octylpalmitat, Mandelöl, Avocadoöl, Jojobaöl, Isostearylisostearat, Octyldodecanol, Dibutyladipat (Cetiol B), Cocossäureglyceride (Myritol 331), Dicaprilylether (Cetiol OE), Isostearylneopentanoat (Ceraphyl 375), C12-15 Alkyllactate (Ceraphyl 41), Dioctylmalat (Ceraphyl 45), sowie die flüssigen Lichtfilter Eusolex® 2292, Eusolex® OCR, Eusolex® 6007, Eusolex® HMS und Eusolex® OS.

Die erfindungsgemäße dermatologische oder kosmetische Zusammensetzung enthält das zuvor beschriebene Konjugat oder eine Kombination der zuvor beschriebenen Konjugate und mindestens einen kosmetisch, pharmazeutisch und/oder dermatologisch verträglichen Träger und/oder Hilfsstoff. Die dermatologische oder kosmetische Zusammensetzung kann das erfindungsgemäße Konjugat natürlich auch in Kombination mit anderen Wirkstoffen, wie z.B. organischen oder anorganischen UVA-und UVB-Filtern, IR- oder VIS-Filtem enthalten. Besonders bevorzugt ist die Kombination mit weiteren UV-Filtern, umfassend die zuvor beschriebenen ungebundenen Lichtfilter, oder deren Gemischen. Dabei liegt das Verhältnis von gebundenen Wirkstoffen (d.h. Wirkstoffe, die an Pigmente gebunden sind) zu ungebundenen Wirkstoffen (d.h. Wirkstoffe, die nicht an Pigmente gebunden sind) bevorzugt im Bereich von 1:10 bis 10:1 und besonders bevorzugt im Bereich von 1:5 bis 5:1. Es können z.B. an Pigmente gebundene Lichtfilter mit ungebundenen Lichtfiltern kombiniert werden.

Die erfindungsgemäße dermatologische oder kosmetische Zusammensetzung enthält das erfindungsgemäße Konjugat, evtl. in Kombination mit weiteren kosmetischen Stoffen, bevorzugt in einer Menge im Bereich von 0,05 bis 30 Gew.-%, besonders bevorzugt in einer Menge im Bereich von 0,5 bis 10 Gew.-% und ganz besonders bevorzugt in einer Menge im Bereich von 0,5 bis 6 Gew.-%, bezogen auf das Gesamtgewicht der dermatologischen oder kosmetischen Zusammensetzung.

### Definitionen der kosmetischen Stoffe:

Beispiele für anorganische UV-Filter umfassen gecoatetes Titandioxid (z.B. Eusolex® T-2000 oder Eusolex® T-AQUA), Zinkoxide (z.B. Sachtotec®), Eisenoxide und Ceroxide. Diese anorganischen UV-Filter werden in der Regel in einer Menge von 0,5 bis 10 Gew.%, vorzugsweise 2 bis 5 Gew.%, in die erfindungsgemäßen dermatologischen oder kosmetischen Zusammensetzungen eingearbeitet.

Beispiele für Träger und Hilfsstoffe umfassen Verdickungsmittel, weichmachende Mittel, Befeuchtungsmittel, grenzflächenaktive Mittel, Emulgatoren, Konservierungsmittel, Mittel gegen Schaumbildung, Parfüms, Fette und Wachse, Lanolin, Treibmittel, Stabilisatoren, Antioxidantien, Bakterizide, Farbstoffe und/oder Pigmente, welche die Formulierung selbst oder die Haut färben, Filmbildner, Geruchsverbesserer, Komplexbildner und andere in der Kosmetik gewöhnlich verwendete Additive.

Als Dispersions- bzw. Solubilisierungsmittel kann ein kosmetisches Öl, ein Wachs oder ein sonstiger Fettkörper, ein Alkohol oder ein Polyol oder Mischungen davon verwendet werden. Zu den besonders bevorzugten Alkoholen oder Polyolen zählen Ethanol, i-Propanol, Propylenglycol, Glycerin und Sorbit.

Als Emulgatoren kommen vorzugsweise bekannte W/O- und daneben auch O/W-Emulgatoren wie z.B. Polyglycerinester, Sorbitanester oder teilveresterte Glyceride in Betracht.

Typische Beispiele für Fette umfassen Glyceride, und als Wachse können z.B. Bienenwachs, Carnaubawachs, Paraffinwachs oder Mikrowachse, gegebenenfalls in Kombination mit hydrophilen Wachsen, verwendet werden.

Als Stabilisatoren können Metallsalze von Fettsäuren, wie z.B. Magnesium-, Aluminium- und/oder Zinkstearat, eingesetzt werden.

Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon.

Verwendbare Filmbildner umfassen Hydrocolloide, wie z.B. Chitosan, mikrokristallines Chitosan oder quaternäres Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich beispielsweise Formaldehydlösungen, p-Hydroxybenzoat oder Sorbinsäure.

Als Perlglanzmittel kommen beispielsweise Glycoldistearinsäureester, wie z.B. Ethylenglycoldistearat, aber auch Fettsäuren und Fettsäuremonoglycolester in Betracht.

Als Farbstoffe können die für kosmetische Zwecke geeigneten und zugelassenen Substanzen verwendet werden, wie sie beispielweise in der Veröffentlichung "Kosmetische Färbemittel" der Farbstoffkommission der Deutschen Forschungsgemeinschaft, veröffentlicht im Verlag Chemie, Weinheim, 1984, zusammengestellt sind.

Als Antioxidantien können beispielsweise Aminosäuren, Imidazole, Peptide, Carotinoide, α-Hydroxysäuren, ungesättigte Fettsäuren, Vitamin A, C und/oder E und geeignete Derivate dieser Stoffe verwendet werden, sowie Zink und dessen Verbindungen (wie z.B. ZnO, ZnSO₄) oder Selen und dessen Verbindungen (wie z.B. Selenmethionin). Bevorzugte Antioxidantien umfassen die zuvor genannten Substanzen mit antioxidativen Eigenschaften, wobei Flavonoide, Coumaranone, Vitamine und BHT bevorzugt sind.

Mischungen von Antioxidantien können ebenfalls in den erfindungsgemäßen dermatologischen und kosmetischen Zusammensetzungen verwendet werden. Bekannte und handelsüblich erhältliche Mischungen umfassen Mischungen, die Lecithin, L-(+)-Ascorbylpalmitat und Zitronen-säure (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder BHT, L-(+)-Ascorbylpalmitat und Zitronensäure (z.B. Oxynex® 2004) als aktive Inhaltsstoffe enthalten.

In einer bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten.

Unter den Flavonoiden sind insbesondere Rutin und Troxerutin bevorzugt. Besonders bevorzugt ist Troxerutin.

Unter den Coumaranonen sind 4,6,3',4'-Tetrahydroxybenzylcoumaranon-3 sowie dessen Salze (Sulfat, Phosphat) bevorzugt.

Die erfindungsgemäße dermatologische oder kosmetische Zusammensetzung kann als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen dermatologischen oder kosmetischen Zusammensetzungen enthalten, insbesondere bevorzugt Vitamin-A-Palmitat, Vitamin C, DL-α-Tocopherol, Tocopherol-E-Acetat, Nicotinsäure, Pantothensäure und Biotin.

Gegebenenfalls können die erfindungsgemäßen dermatologischen oder kosmetischen Zusammensetzungen auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden.

Solche können sowohl pflanzliche Extrakte als auch synthetische Selbstbräuner, wie z.B. Dihydroxyaceton oder α-Ketole sowie Erythrolose, sein.

Anwendungsformen der erfindungsgemäßen dermatologischen oder kosmetischen Zusammensetzungen umfassen: Suspensionen, Emulsionen, Fettstifte, Pasten, Salben, Cremes oder Milch (O/W, W/O, O/W/O, W/O/W), Lotionen, Puder, Seifen, tensidhaltige Reinigungspräparate, Öle, Aerosole, Sprays sowie ölig-alkoholische, ölig-wässrige oder wässrig-alkoholische Gele bzw. Lösungen. Weitere Anwendungsformen sind z.B. Sticks, Shampoos und Duschbäder.

Die wässrige Phase der erfindungsgemäßen dermatologischen oder kosmetischen Zusammensetzungen enthält bevorzugt Alkohole, Diole oder Polyole, sowie Ether, vorzugsweise Ethanol, Isopropanol, 1,2-Propandiol, Propylenglykol, Glycerin, Ethylenglykol, Ethylenglykolmonoethyl- oder -monobutylether oder analoge Produkte, ferner ein oder mehrere Verdickungsmittel, wie z.B. Siliciumdioxid, Aluminiumsilikate, Polysaccharide bzw. deren Derivate, z.B. Hyaluronsäure, Xanthangummi, Hydroxypropylemethylcellulose, oder ein Polyacrylat aus der Gruppe der sogenannten Carbopole.

Verwendbare Ölkomponenten umfassen die zuvor beschriebenen Ölkomponenten.

Salben, Pasten, Cremes und Gele können die üblichen Trägerstoffe enthalten, wie z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, wie z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe, wie z.B. Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe, enthalten.

Suspensionen können die üblichen Trägerstoffe, wie z.B flüssige Verdünnungsmittel, z.B. Wasser, Ethanol oder Propylenglykol, Suspendiermittel, wie z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe, enthalten.

Seifen können die üblichen Tragerstoffe, wie z.B. Alkalisalze von Fettsäuren, Salze von Fettsäurehalbestern, Fettsäureeiweißhydrolysate, Isothionate, Lanolin, Fettalkohol, Pflanzenöle, Pflanzenextrakte, Glycerin, Zucker oder Gemische dieser Stoffe, enthalten.

Tensidhaltige Reinigungsprodukte können die üblichen Trägerstoffe, wie z.B. Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe, enthalten.

Gesichts- und Körperöle können die üblichen Trägerstoffe, wie z.B. synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe, enthalten. Bevorzugte Beispiele für solche Trägerstoffe umfassen die zuvor genannten Ölkomponenten.

In einer bevorzugten Ausführungsform ist die erfindungsgemäße dermatologische oder kosmetische Zusammensetzung eine Emulsion, welche als Schutzcreme oder -milch vorliegt und außer dem erfindungsgemäßen Konjugat Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf der Basis von natürlichen oder synthetischen Ölen und Wachsen, Lanolin, Fettsäureestern, insbesondere Triglyceriden von Fettsäuren, oder ölig-alkoholische Lotionen auf der Basis eines Alkohols, wie Ethanol, oder eines Glycols, wie Propylenglycol, und/oder eines Polyols, wie Glycerin, und Ölen, Wachsen und Fettsäureestern, wie Triglyceriden von Fettsäuren, dar.

Die erfindungsgemäße dermatologische oder kosmetische Zusammensetzung kann auch als alkoholisches Gel vorliegen, welches einen oder mehrere Alkohole oder Polyole, wie Ethanol, Propylenglycol oder Glycerin, und ein Verdickungsmittel, wie Kieselerde, umfaßt. Die ölig-alkoholischen Gele können außerdem ein natürliches oder synthetisches Öl oder Wachs enthalten.

Die festen Stifte können natürliche oder synthetische Wachse und Öle, Fettalkohole, Fettsäuren, Fettsäureester, Lanolin und andere Fettkörper enthalten.

Ist die erfindungsgemäße dermatologische oder kosmetische Zusammensetzung als Aerosol konfektioniert, werden in der Regel die üblichen Treibmittel, wie Alkane, Fluoralkane und Chlorfluoralkane, verwendet.

Weitere typisch kosmetische Anwendungsformen umfassen Lippenstifte, Lippenpflegestifte, Rouge, Puder-, Emulsions- und Wachs-Make up sowie Prä-Sun- und After-Sun-Präparate.

Alle Träger und Hilfsstoffe, die in den erfindungsgemäßen dermatologischen oder kosmetischen Zusammensetzungen verwendet werden können, sind entweder bekannt und handelsüblich erhältlich oder können nach bekannten Verfahren synthetisiert werden.

Die erfindungsgemäße dermatologische oder kosmetische Zusammensetzung kann die zuvor genannten Träger und Hilfsstoffe jeweils in einer Menge im Bereich von 0,001 bis 30 Gew.-%, bevorzugt in einer Menge im Bereich von 0,05 bis 20 Gew.-% und besonders bevorzugt in einer Menge im Bereich von 1 bis 10 Gew.-% enthalten.

Die erfindungsgemäße dermatologische oder kosmetische Zusammensetzung kann mit Hilfe von Verfahren hergestellt werden, die dem Fachmann bekannt sind.

Das erfindungsgemäße Konjugat kann ebenfalls zum Schutz der Haut, insbesondere zum Schutz der Langerhans-Zellen in der Haut, zum Schutz der DNA oder zur Immunprotektion verwendet werden.

Das erfindungsgemäße Konjugat kann auch außerhalb der Kosmetik verwendet werden, z.B. zur Herstellung eines Lackes oder einer Sicherheitsmarkierung. In diesem Fall kann z.B. die Gruppe R¹ in der zuvor genannten allgemeinen Formel eine fluoreszierende Gruppe sein, die durch UV-Licht angeregt werden kann.

Die folgenden Beispiele erläutern die Erfindung. Die eingesetzten Ausgangsmaterialien sind entweder handelsüblich erhältlich oder können in bekannter Weise synthetisiert werden.

### Beispiele

### Beispiel 1 Herstellung eines Eusolex® 9020/Monospher® 100-Konjugats

Ein Eusolex® 9020/Monospher® 100-Konjugat wurde entsprechend dem folgenden Reaktionsschema hergestellt.

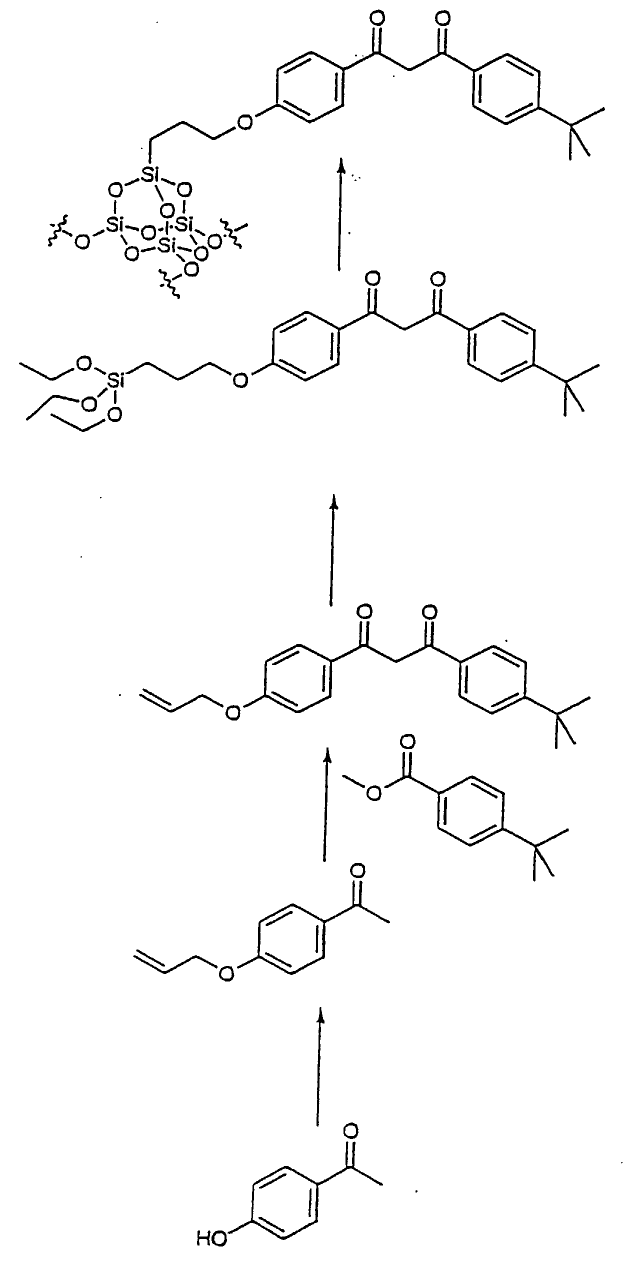

### Herstellung von 4-(2-Propenyloxy)-acetophenon (1. Durchführung)

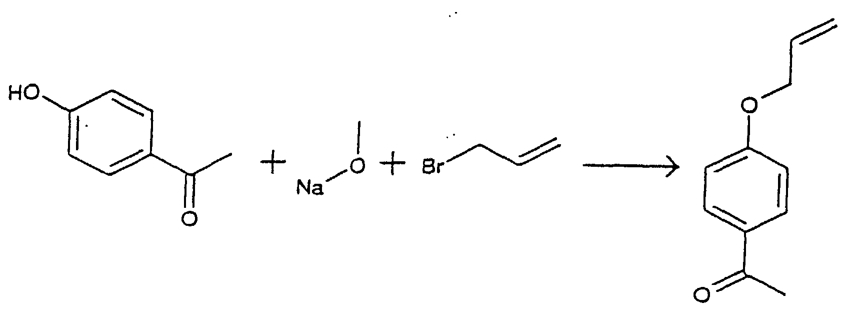

Die zur Durchführung der Umsetzung verwendete Apparatur wurde mit Stickstoff gespült, um die Luft in der Apparatur zu verdrängen. Dann wurden 116 g Methanol und 16,67 g 4-Hydroxyacetophenon in die Apparatur gegeben. Das Gemisch wurde gerührt, bis eine homogene Lösung erhalten wurde. Dann wurden innerhalb von 10 Minuten 24,85 g Natriummethylat (30%ige Lösung in Methanol) zugetropft, wobei unter exothermer Reaktion eine rötliche Lösung erhalten wurde. Danach wurde die Lösung auf 52°C erwärmt, und dann wurden über einen Zeitraum von 12 Minuten 16,31 g 3-Brom-1-propen zugetropft. Das Reaktionsgemisch wurde 15 Stunden lang bei einer Temperatur von 63 bis 64°C gehalten.

Die gelbe Reaktionslösung wurde auf 32°C abgekühlt, und der Methanol wurde am Rotationsverdampfer abgezogen. Das erhaltene teilkristalline Konzentrat wurde mit 120 g Toluol und 50 g entionisiertem Wasser versetzt. Danach wurde 10 Minuten lang nachgerührt.

Nach der Phasentrennung wurde die organische Phase am Rotationsverdampfer azeotrop zu einem gelben Öl konzentriert.

Ausbeute:
Rohprodukt: 22,89 g

Das Rohprodukt wurde bei 116 bis 118°C Luftbadtemperatur im Vakuum (0,7 bis 0,9 mbar) destilliert, wobei 18,71 g Destillat erhalten wurden. Das Destillat wurde nochmals bei 115°C im Vakuum (0,7 bis 0,9 mbar) destilliert, wobei 18,10 g 4-(2-Propenyloxy)-acetophenon erhalten wurden.

### Herstellung von 4-(2-Propenyloxy)-acetophenon (2. Durchführung)

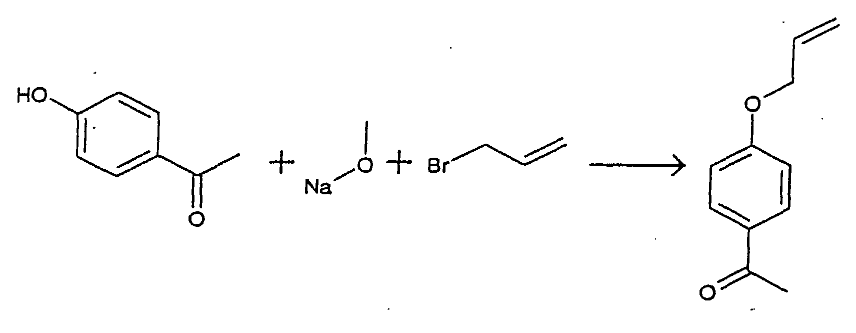

Die zur Durchführung der Umsetzung verwendete Apparatur wurde mit Stickstoff gespült, um die Luft in der Apparatur zu verdrängen. Dann wurden 116 g Methanol und 16,67 g 4-Hydroxyacetophenon in die Apparatur gegeben. Das Gemisch wurde gerührt, bis eine homogene Lösung erhalten wurde. Dann wurden innerhalb von 8 Minuten 27 g Natriummethylat (30%ige Lösung in Methanol) zugetropft, wobei unter exothermer Reaktion eine rötliche Lösung erhalten wurde. Danach wurde die Lösung auf 55°C erwärmt, und dann wurden über einen Zeitraum von 16 Minuten 17,77 g 3-Brom-1-propen zugetropft. Das Reaktionsgemisch wurde 14,75 Stunden lang bei einer Temperatur von 63 bis 64°C gehalten.

Die gelbe Reaktionslösung wurde auf 28°C abgekühlt, und der Methanol wurde am Rotationsverdampfer abgezogen. Das erhaltene teilkristalline Konzentrat wurde mit 120 g Toluol und 50 g entionisiertem Wasser versetzt. Danach wurde 5 Minuten lang nachgerührt.

Nach der Phasentrennung wurde die organische Phase mit 20 ml Natronlauge (1-molar) und zweimal mit je 25 g entionisiertem Wasser gewaschen und dann am Rotationsverdampfer azeotrop zu einem gelben Öl konzentriert.

Ausbeute:
Rohprodukt: 20,1 g

Das Rohprodukt wurde bei 115 bis 116°C Luftbadtemperatur im Vakuum (0,6 bis 0,7 mbar) destilliert, wobei 18,56 g 4-(2-Propenyloxy)-acetophenon erhalten wurden.

### Herstellung eines kopplungsfähigen Analogons von Eusolex® 9020 (1. Durchführung)

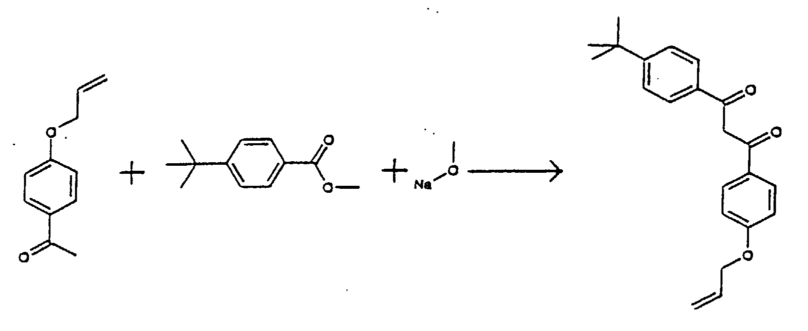

Die zur Durchführung der Umsetzung verwendete Apparatur wurde mit Stickstoff gespült, um die Luft in der Apparatur zu verdrängen. Dann wurden 268 g Cyclohexan vorgelegt und innerhalb von 15 Minuten auf 48°C erwärmt. Danach wurden innerhalb von 2 Minuten 27,03 g Natriummethylat (30%ige Lösung in Methanol) zugetropft. Die Emulsion wurde auf 64°C erwärmt, und dann wurde über einen Zeitraum von 9 Minuten eine Lösung von 27,9 g 4-t-Butylbenzoesäuremethylester in 35 g Cyclohexan zugetropft. Das Reaktionsgemisch wurde 20 Minuten lang gerührt, bis die Reaktionstemperatur 80,5°C erreicht hatte. Dabei wurden 40 ml im Wasserabscheider gebildetes Cyclohexan/Methanol-Gemisch portioniert abgelassen. Dann wurde über einen Zeitraum von 60 Minuten eine Lösung von 17,64 g 4-(2-Propenyloxy)-acetophenon in 36 g Cyclohexan zugetropft. Während dieser Zeit und in den darauffolgenden 60 Minuten wurden in etwa 20-minütigem Abstand jeweils 10 bis 15 ml Cyclohexan/Methanol-Gemisch (insgesamt 70 ml) abgelassen. Die Reaktionstemperatur lag bei 75 bis 80°C. Danach wurden innerhalb von weiteren 2,5 Stunden 84 g Cyclohexan zugetropft. Gelegentlich wurde ein Cyclohexan/Methanol-Gemisch (insgesamt 80 ml) portioniert abgelassen. Der Ansatz wurde insgesamt 7,5 Stunden lang kräftig gerührt.

Die Suspension wurde dann auf 70°C abgekühlt, und danach wurde ein Gemisch aus 36 g Essigsäure (100%ig) und 30 g entionisiertem Wasser zugetropft und 10 Minuten lang nachgerührt.

Nach der Phasentrennung bei 50°C wurde die organische Phase zweimal mit 50 bzw. 20 g entionisiertem Wasser gewaschen und am Rotationsverdampfer azeotrop zu einem rotbraunen Öl konzentriert.

### Ausbeute Rohprodukt: 42,9 g

Das Rohprodukt wurde bei 118 bis 133°C Luftbadtemperatur im Vakuum (0,5 bis 0,7 mbar) destilliert, wobei 13,36 g Destillat und 29,22 g Destillationsrückstand erhalten wurden.

Der Destillationsrückstand wurde unter Erwärmen (50 bis 60°C) in 50 g 2-Propanol gelöst, über Watte filtriert und unter Rühren abgekühlt. Nach der Zugabe von Impfkristallen erfolgte die Kristallisation (30 bis 40°C).

Die Suspension wurde nach 60 Minuten Rührdauer bei Raumtemperatur in weiteren 60 Minuten langsam auf 4 bis 6°C abgekühlt. Das Kristallisat wurde isoliert, portioniert mit kaltem 2-Propanol gewaschen und trockengesaugt. Das Trocknen erfolgte im Vakuumtrockenschrank bei 35°C.

Ausbeute:
Kristallisat (kopplungsfähiges Eusolex® 9020-Analogon): 23,89 g
Schmelzpunkt des Kristallisats: 65 bis 67°C

### Herstellung eines kopplungsfähigen Analogons von Eusolex® 9020 (2. Durchführung)

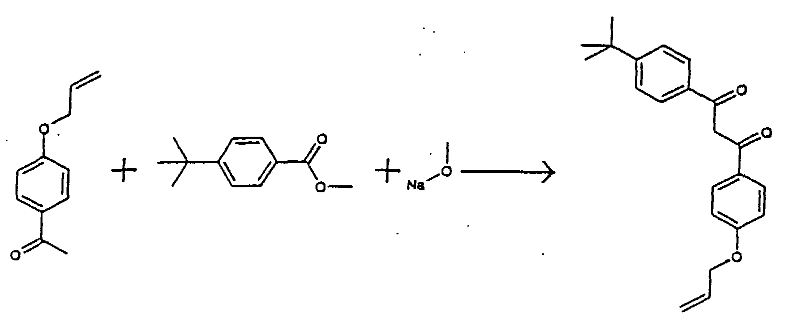

Die zur Durchführung der Umsetzung verwendete Apparatur wurde mit Stickstoff gespült, um die Luft in der Apparatur zu verdrängen. Dann wurden 119 g Cyclohexan vorgelegt und innerhalb von 15 Minuten auf 40°C erwärmt. Danach wurden innerhalb von 2 bis 3 Minuten 13,5 g Natriummethylat (30%ige Lösung in Methanol) zugetropft. Die Emulsion wurde auf 70°C erwärmt, und dann wurde über einen Zeitraum von 8 Minuten eine Lösung von 14,02 g 4-t-Butylbenzoesäuremethylester in 25 g Cyclohexan zugetropft. Das Reaktionsgemisch wurde 12 Minuten lang bei 79°C gerührt. Dabei wurden zweimal je 20 ml im Wasserabscheider gebildetes Cyclohexan/Methanol-Gemisch portioniert abgelassen. Dann wurde über einen Zeitraum von 25 Minuten eine Lösung von 8,81 g 4-(2-Propenyloxy)-acetophenon in 25 g Cyclohexan zugetropft. Das inhomogene Reaktionsgemisch wurde bei 95 bis 100°C Badtemperatur kräftig gerührt, wobei die Temperatur des Reaktionsgemisches innerhalb von 75 Minuten auf 75°C abfiel. Nach erneutem Ablassen des Cyclohexan/Methanol-Gemisches stieg die Temperatur des Reaktionsgemisches auf 80°C an. Der Ansatz wurde weitere 4,75 Stunden bei 79 bis 82°C kräftig gerührt. Während dieser Zeit wurden fünfmal ein Cyclohexan/Methanol-Gemisch abgelassen und 126 g Cyclohexan zugetropft.

Die Suspension wurde dann auf 70°C abgekühlt, und danach wurde ein Gemisch aus 18 g Essigsäure (100%ig) und 15 g entionisiertem Wasser zugetropft und 10 Minuten lang nachgerührt.

Nach der Phasentrennung bei 50°C wurde die organische Phase zweimal mit 50 bzw. 20 g entionisiertem Wasser gewaschen und am Rotationsverdampfer azeotrop zu einem braunen Öl konzentriert.

### Ausbeute Rohprodukt: 21,4 g

Das Rohprodukt wurde bei 115 bis 133°C Luftbadtemperatur im Vakuum (0,6 bis 0,8 mbar) destilliert, wobei 5,89 g Destillat und 15,30 g Destillationsrückstand erhalten wurden.

Der Destillationsrückstand wurde unter Erwärmen (50 bis 60°C) in 30 g 2-Propanol gelöst, über Watte filtriert und unter Rühren abgekühlt. Nach der Zugabe von Impfkristallen erfolgte die Kristallisation (30 bis 40°C).

Die Suspension wurde nach 60 Minuten Rührdauer bei Raumtemperatur in weiteren 45 Minuten langsam auf 10°C abgekühlt. Das Kristallisat wurde isoliert, portioniert mit kaltem 2-Propanol gewaschen und trockengesaugt. Das Trocknen erfolgte im Vakuumtrockenschrank bei 35°C.

Ausbeute:
Kristallisat (kopplungsfähiges Eusolex® 9020-Analogon): 11,35 g
Schmelzpunkt des Kristallisats: 66,5 bis 68°C

### Umkristallisation

33,5 g der zuvor erhaltenen Kristallisate wurden unter Erwärmen (60 bis 70°C) in 84 g 2-Propanol gelöst. Die Lösung wurde über Watte abfiltriert und unter Rühren abgekühlt. Nach der Zugabe von Impfkristallen erfolgte die Kristallisation (44 bis 45°C).

Das Kristallisat wurde nach 2,5 Stunden Rührdauer bei Raumtemperatur isoliert, portioniert mit 2-Propanol gewaschen, trockengesaugt und im Vakuumtrockenschrank bei 35°C getrocknet.

Ausbeute:
Kristallisat: 29,90 g (Schmelzpunkt: 67,1°C)

### Herstellung eines silanisierten Eusolex® 9020-Analogons

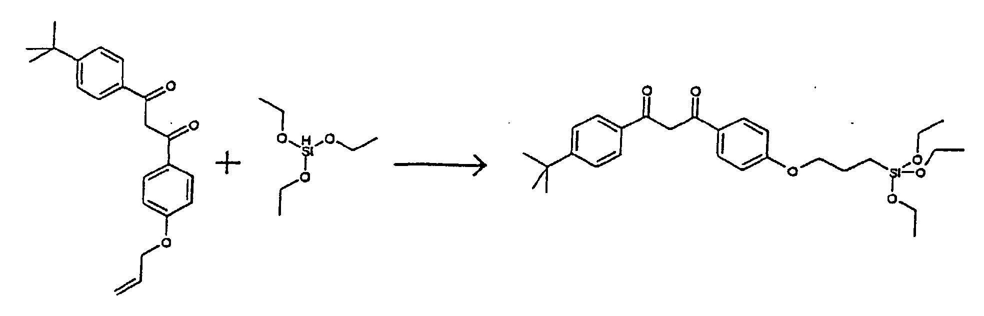

Die zur Durchführung der Umsetzung verwendete Apparatur wurde mit Stickstoff gespült, um die Luft in der Apparatur zu verdrängen. Dann wurden 11,21 g des Kristallisats in 7,98 g THF in der Apparatur gelöst. Danach wurden 16,38 g frisch destilliertes Triethoxysilan (HSi(OC₂H₅)₃) und etwa 2,5 bis 3 mg Hexachloroplatin(IV)-säure-Hexahydrat (40% Pt) zugegeben. Durch die exotherme Reaktion stieg die Temperatur der Lösung auf 30°C an.

THF und überschüssiges Triethoxysilan wurden bei 50°C Luftbadtemperatur im Vakuum (10 bis 11 mbar) abgezogen. Das Destillat bestand aus Triethoxysilan. Das Rohprodukt wurde eine weitere Stunde lang bis 55°C im Vakuum (0,8 bis 1,0 mbar) konzentriert.

Ausbeute Rohprodukt: 16,02 g

### Kopplung des silanisierten Eusolex® 9020-Analogons an Monospher® 100

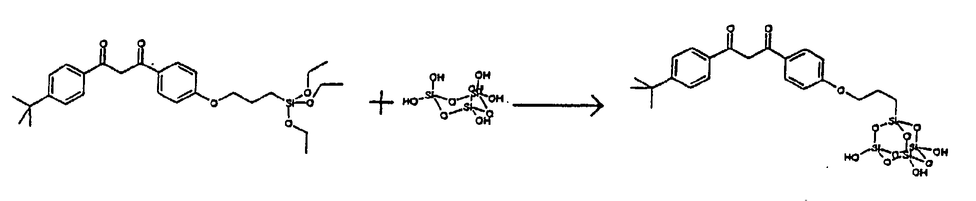

Eine 10%ige Dispersion von 100 g Monospher® 100 in ca. 1 I Ethanol wurde unter Stickstoffschutz auf Rückflußtemperatur erwärmt.

16,02 g des silanisierten Eusolex® 9020-Analogons wurden in 64 g Ethanol gelöst und in einen Tropftrichter eingefüllt.

In der Siedehitze wurden innerhalb von 70 Minuten etwa 50% der Lösung des silanisierten Eusolex® 9020-Analogons zugetropft. Die erhaltene schwach gelbliche Dispersion wurde 5,5 Stunden lang bei Rückfluß gerührt. Danach wurde innerhalb von 60 Minuten die restliche Lösung zugetropft. Der Ansatz wurde über Nacht 15 Stunden lang nachgerührt.

Die Dispersion wurde am Rotationsverdampfer zu einem festen Rückstand eingeengt. Der Rückstand wurde dann 68 Stunden lang bei 40°C im Vakuumtrockenschrank getrocknet.

Ausbeute an Eusolex® 9020/Monospher® 100-Konjugat: 106,9 g

### Beispiel 2 Herstellung eines Eusolex® 9020/Monospher® 25-Konjugats

Ein Eusolex® 9020/Monospher® 25-Konjugat wurde entsprechend dem folgenden Reaktionsschema hergestellt.

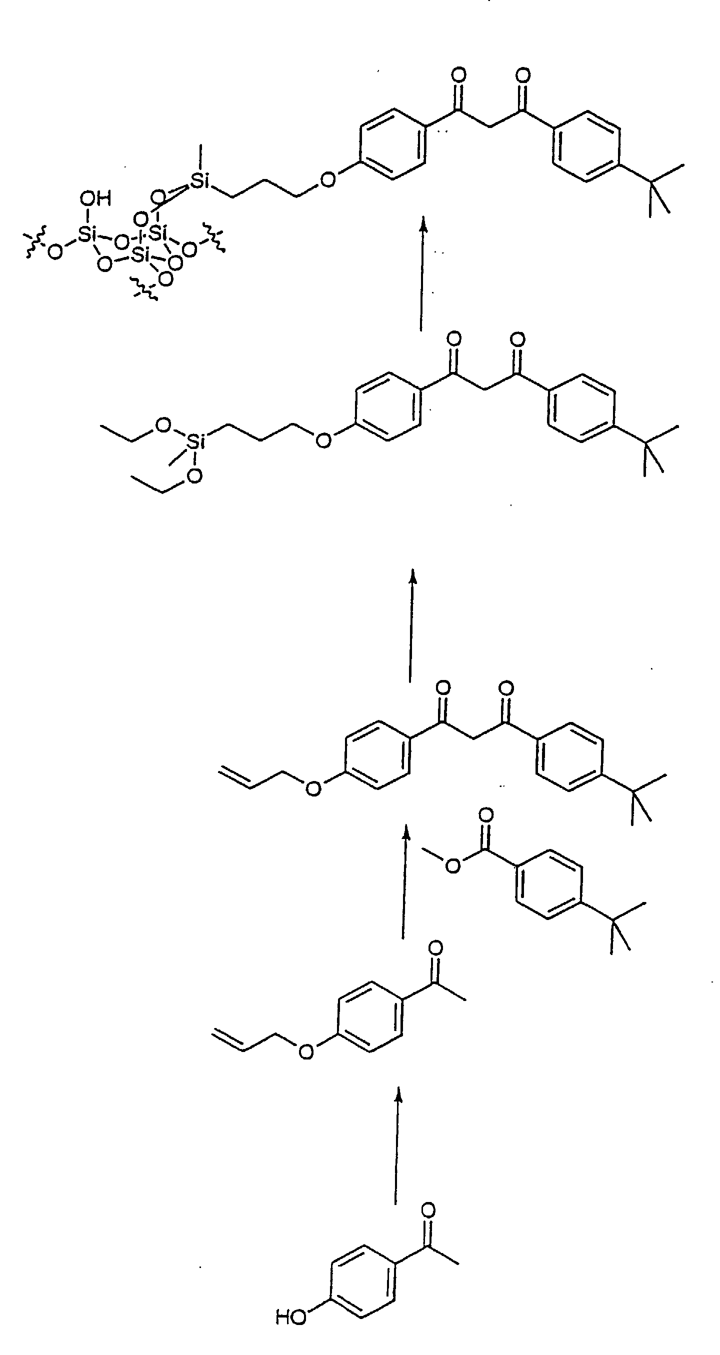

### Herstellung von 4-(2-Propenyloxy)-acetophenon

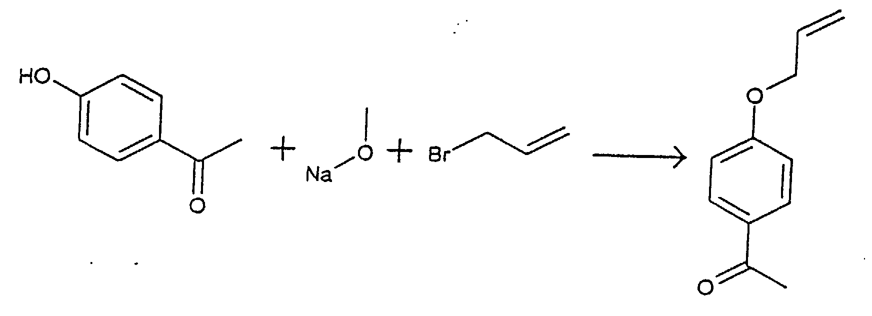

Die zur Durchführung der Umsetzung verwendete Apparatur wurde mit Stickstoff gespült, um die Luft in der Apparatur zu verdrängen. Dann wurden 116 g Methanol und 16,67 g 4-Hydroxyacetophenon in die Apparatur gegeben. Das Gemisch wurde gerührt, bis eine homogene Lösung erhalten wurde. Dann wurden innerhalb von 15 Minuten 77,8 g Natriummethylat (30%ige Lösung in Methanol) zugetropft, wobei unter exothermer Reaktion eine rötliche Lösung erhalten wurde. Danach wurde die Lösung auf 56°C erwärmt, und dann wurden über einen Zeitraum von 39 Minuten 50,6 g 3-Brom-1-propen zugetropft. Das Reaktionsgemisch wurde 21 Stunden lang bei einer Temperatur von 63 bis 65°C gehalten.

Die gelbe Reaktionslösung wurde auf 40°C abgekühlt, und der Methanol wurde am Rotationsverdampfer abgezogen. Das erhaltene teilkristalline Konzentrat wurde mit 200 g Toluol und 100 g entionisiertem Wasser versetzt. Danach wurde 5 Minuten lang nachgerührt.

Nach der Phasentrennung wurde die organische Phase mit 50 ml Natronlauge (1-molar) und zweimal mit je 50 g entionisiertem Wasser gewaschen, über Watte filtriert und dann am Rotationsverdampfer azeotrop zu einem gelben Öl konzentriert.

Ausbeute Rohprodukt: 60,9 g

60,7 g des Rohproduktes wurden über eine 10 cm-Vigreuxkolonne im Vakuum (0,8 bis 1,4 mbar) fraktioniert destilliert. Die Destillationsdauer betrug 3 Stunden. Dabei wurden 50,89 g 4-(2-Propenyloxy)-acetophenon erhalten.

### Herstellung eines kopplungsfähigen Analogons von Eusolex® 9020

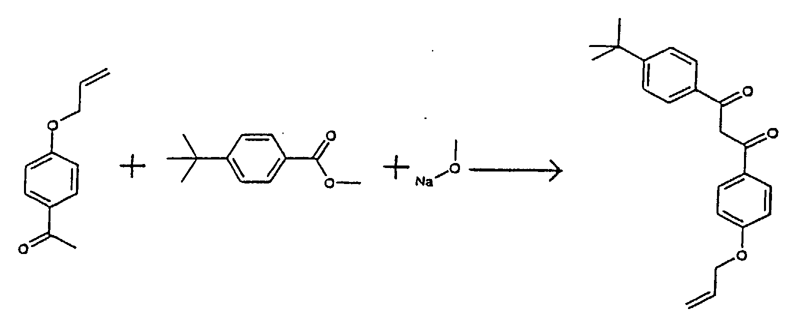

Die zur Durchführung der Umsetzung verwendete Apparatur wurde mit Stickstoff gespült, um die Luft in der Apparatur zu verdrängen. Dann wurden 764 g Cyclohexan vorgelegt und innerhalb von 14 Minuten auf 50°C erwärmt. Danach wurden innerhalb von 6 Minuten 77 g Natriummethylat (30%ige Lösung in Methanol) zugetropft. Die Emulsion wurde auf 64°C erwärmt, und dann wurde über einen Zeitraum von 6 Minuten eine Lösung von 79,6 g 4-t-Butylbenzoesäuremethylester in 100 g Cyclohexan zugetropft. Das Reaktionsgemisch wurde 70 Minuten lang gerührt, bis die Reaktionstemperatur 80°C erreicht hatte. Dabei wurden 220 ml im Wasserabscheider gebildetes Cyclohexan/Methanol-Gemisch portioniert abgelassen. Dann wurde über einen Zeitraum von 50 Minuten eine Lösung von 50,24 g 4-(2-Propenyloxy)-acetophenon in 100 g Cyclohexan zugetropft. Während dieser Zeit und in den darauffolgenden 4 Stunden wurden portioniert zwischen 40 und 80 ml Cyclohexan/Methanol-Gemisch (insgesamt 400 ml) abgelassen. Die Reaktionstemperatur lag bei 75 bis 80°C. Während der Reaktionsdauer wurden dreimal je 120 g Cyclohexan zugetropft. Der Ansatz wurde weitere 16 Stunden lang gerührt, wobei die Reaktionstemperatur bei 79,5 bis 81°C gehalten wurde.

Die Suspension wurde dann auf 70°C abgekühlt, und danach wurde ein Gemisch aus 102,8 g Essigsäure (100%ig) und 85 g entionisiertem Wasserzugetropft und 40 Minuten lang nachgerührt.

Nach der Phasentrennung bei 40°C wurde die organische Phase dreimal mit je 50 ml Natronlauge (1-molar) und zweimal mit 100 bzw. 50 g entionisiertem Wasser gewaschen und am Rotationsverdampfer azeotrop zu einem rotbraunen Öl konzentriert.

Ausbeute Rohprodukt: 115,7 g

Das Rohprodukt wurde unter Erwärmen in 145 g 2-Propanol gelöst, über Watte filtriert und unter Rühren abgekühlt. Nach der Zugabe von Impfkristallen erfolgte die Kristallisation (34°C).

Die Suspension wurde nach 3 Stunden Rührdauer bei 28°C in weiteren 60 Minuten langsam auf 10°C abgekühlt. Das Kristallisat wurde isoliert, portioniert mit kaltem 2-Propanol gewaschen und trockengesaugt. Das Trocknen erfolgte im Vakuumtrockenschrank bei 35°C.

Ausbeute:
Kristallisat (kopplungsfähiges Eusolex® 9020-Analogon): 67,8 g
Schmelzpunkt des Kristallisats: 66,8°C

67,8 g Kristallisat wurden unter Erwärmen auf 60°C in 156 g 2-Propanol gelöst. Die Lösung wurde unter Rühren abgekühlt. Nach der Zugabe von Impfkristallen erfolgte die Kristallisation (44 bis 45°C).

Die Suspension wurde nach 2 Stunden Rührdauer bei 26°C in weiteren 60 Minuten langsam auf 10°C abgekühlt. Das Kristallisat wurde isoliert, portioniert mit kaltem 2-Propanol gewaschen, trockengesaugt und bei 35°C im Vakuumtrockenschrank getrocknet.

Ausbeute Kristallisat: 65,0 g (Schmelzpunkt: 67,2°C)

### Herstellung eines silanisierten Eusolex® 9020-Analogons

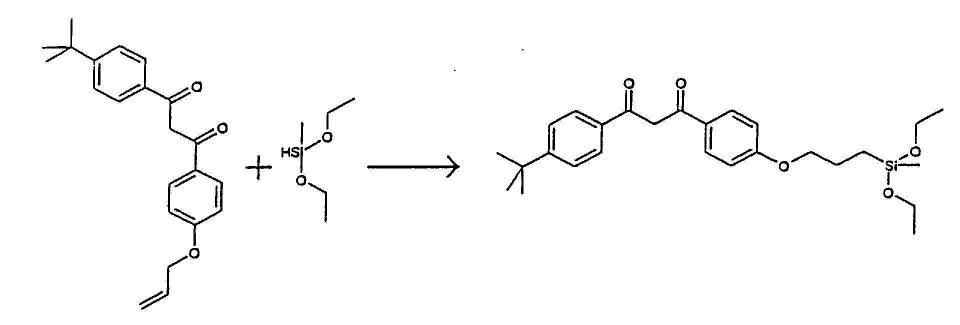

Die zur Durchführung der Umsetzung verwendete Apparatur wurde mit Stickstoff gespült, um die Luft in der Apparatur zu verdrängen. Dann wurden 17,83 g des Kristallisats in 9,6 g THF in der Apparatur gelöst. Der schwach gelblichen Lösung wurden 14,82 g Methyldiethoxysilan (HSiCH₃(OC₂H₅)₂) und etwa 4 bis 4,5 mg Hexachloroplatin(IV)-säure-Hexahydrat (40% Pt) zugegeben. Durch die exotherme Reaktion stieg die Temperatur der Lösung auf 30°C an. Der Ansatz wurde über Nacht nachgerührt.

THF und überschüssiges Methyldiethoxysilan wurden bei 40°C Luftbadtemperatur im Vakuum (0,7 bis 1,5 mbar) abgezogen. Das Rohprodukt wurde bei gleicher Temperatur noch 0,75 Stunden entgast.

Ausbeute Rohprodukt: 24,5 g

### Kopplung des silanisierten Eusolex® 9020-Analogons an Monospher® 25

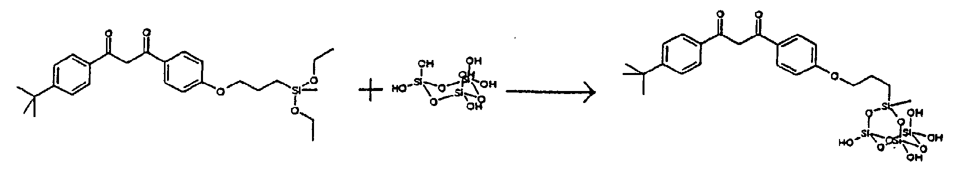

248,5 g einer Dispersion von Monospher® 25 wurden am Rotationsverdampfer durch Abdestillieren von Wasser auf 182 g konzentriert, in einen 2 I-Vierhalskolben überführt und mit 782 g Ethanol auf ca. 1,2 I Volumen aufgefüllt.

Über eine Destillationsbrücke wurden in 8 Stunden 2530 g (ca. 3,15 bis 3,2 l) Ethanol abdestilliert. Das Volumen der Dispersion wurde durch Zugabe von 2347 g frischem Ethanol (Wassergehalt: 0,06%) über Tropftrichter gehalten.

Dem ablaufenden Destillat wurde nach jeweils 1 I eine Probe zur Wasserbestimmung entnommen (Wassergehalt: 5,0, 1,8 und 1,0%).

Mit Beendigung der Destillation wurde das Volumen auf ca. 1000 ml konzentriert.

Die ethanolische Dispersion von Monospher® 25 zur Silanisierung wurde unter Stickstoffschutz auf Rückflußtemperatur erwärmt.

Das silanisierte Eusolex® 9020-Analogon wurde in 90 ml Ethanol gelöst, in einen Tropftrichter eingefüllt und innerhalb von 6 Stunden in der Siedehitze zugetropft.

Der Ansatz wurde 23 Stunden lang unter Rückfluß und anschließend 42 Stunden lang abkühlend nachgerührt.

Schwebeteilchen wurden durch Filtration über Watte entfernt, und die Dispersion (862 g) wurde am Rotationsverdampfer auf einen Trockengehalt zwischen 20 und 40% konzentriert.

Das rötliche Konzentrat enthält theoretisch 99,7 g an Monospher® 25 gebundenes Eusolex® 9020. Dies entspricht einem Trockengehalt von 33,8%. Das molare Verhältnis von Eusolex® 9020 zu Monospher® 25 beträgt 96,24% : 3,76%.

Danach wurden zu Testzwecken jeweils 10 g ethanolisches Konzentrat in Rundkölbchen überführt, mit 7,88 g der im folgenden angegebenen Öle versetzt und am Rotationsverdampfer möglichst lösungsmittelfrei konzentriert. Es sollten 11,26 g Dispersionen mit einem Trockengehalt von 30% erhalten werden.

| Öl | Handelsname | Ausbeute (g) | Gehalt ¹ (%) | Konsistenz ² |
|---|---|---|---|---|
| Butylenglycol-dicaprylat/dicaprinat | Miglyol 8810 | 11,70 | 28,9 | inhomogene Lösung |
| Caprylsäure/ Caprinsäure-Triglyceride | Miglyol 812 Neutralöl | 11,69 | 28,9 | inhomogene Lösung |
| Butylenglycol | 1,3-Butandiol | 11,75 | 28,8 | Lösung/ Emulsion |
| Cocossäure-glyceride | Myritol 331 | 12,00 | 28,2 | Lösung |
| C12-15 Alkyl-benzoat | Finsolv TN | 12,52 | 27,0 | inhomogene Lösung |
| Octylpalmitat | Ceraphyl 368 | 12,06 | 28,0 | inhomogene Lösung |

1) Durch restliches Ethanol verringert sich der Gehalt bei scheinbar höherer Dispersionsausbeute.
2) Bezieht sich auf das Zutropfen der Öle zum ethanolischen Konzentrat.

Die Hauptmenge des ethanolischen Konzentrats (219,5 g) wurde in einen Becherkolben überführt und am Rotationsverdampfer temperiert (Wasserbadtemperatur: 50 bis 55°C). Innerhalb eines Zeitraums von 12 bis 15 Minuten wurden dann 173 g Finsolv TN eingezogen.

Die trübe, homogene Dispersion wurde bis zur Gewichtskonstanz konzentriert; dabei bildete sich zwischenzeitlich eine klare "Lösung".

Ausbeute: 268 g gelbe Paste mit einem theoretischen Feststoffanteil von 74,2 g (27,7%) Eusolex® 9020 gebunden an Monospher® 25.

### Beispiel 3 Herstellung eines BHT/Monospher® 100-Konjugats

Ein BHT/Monospher® 100-Konjugat wurde entsprechend dem folgenden Reaktionsschema hergestellt.

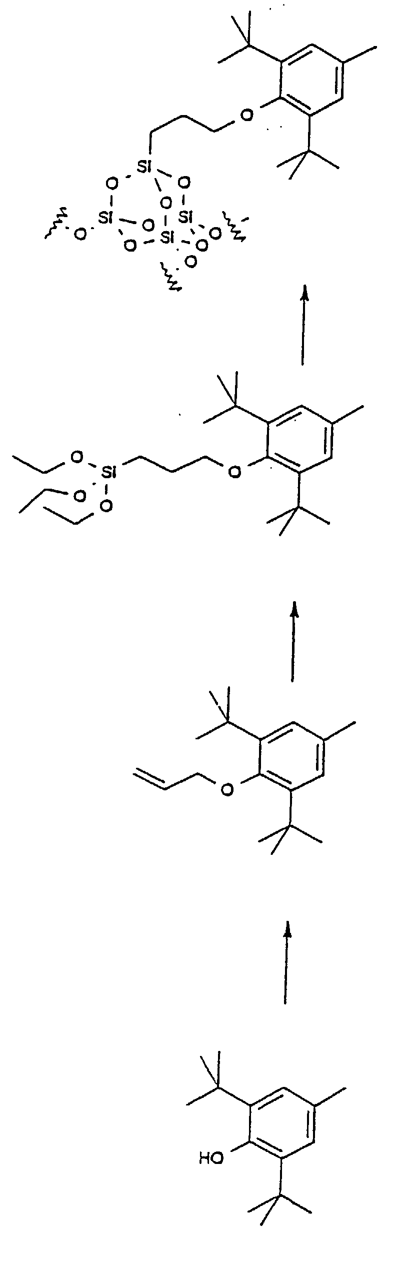

### Herstellung von 2,6-Di-tert-Butyl-4-methyl-1-(3-propen-1-yl-)oxy-benzol

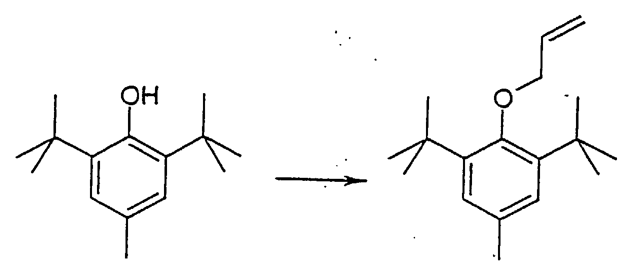

Analog zur Vorschrift von Beispiel 1 wurden 53 g BHT (2,6-Di-tert-Butyl-4-methyl-phenol) mit 35,5 g 3-Brom-1-propen und 54 g Natriummethylat (30%ige Lösung in Methanol) umgesetzt.

Nach Destillation im Vakuum wurden 46,8 g 2,6-Di-tert-Butyl-4-methyl-1-(3-propen-1-yl-)oxy-benzol erhalten, dessen physikalische Daten mit den für die gewünschte Verbindung (CAS-No.: [1516-98-9]) publizierten identisch waren.

### Herstellung des silanisierten Zwischenproduktes

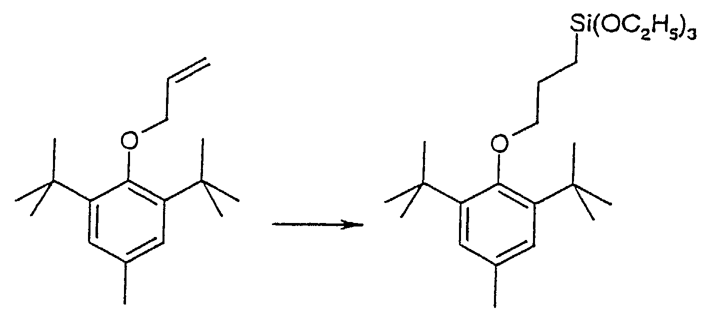

8,85 g 2,6-Di-tert-Butyl-4-methyl-1-(3-propen-1-yl-)oxy-benzol wurden analog zur Vorschrift von Beispiel 1 in THF mit 16,4 g Triethoxysilan umgesetzt. Dazu wurden 2 mg Hexachloroplatin(VI)säure-Hexahydrat als Katalysator eingesetzt.

Es wurden 14,8 g Rohprodukt erhalten.

### Kopplung des silanisierten Zwischenproduktes an Monospher® 100

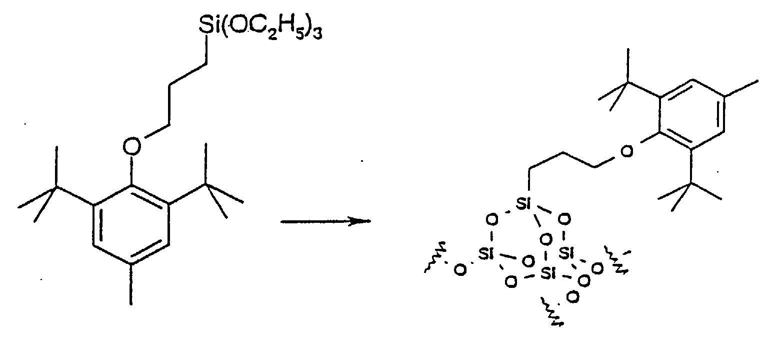

Analog zur Vorschrift von Beispiel 1 wurde eine 10%ige Suspension von 100 g Monospher® 100 in 1 I Ethanol mit den 14,8 g Rohprodukt aus der vorhergehenden Synthese umgesetzt.

Ausbeute an BHT/Monospher® 100-Konjugat: 104,2 g

### Beispiel 4 Herstellung eines Methylparaben/Monospher® 25-Konjugats

Ein Methylparaben/Monospher® 25-Konjugat wurde entsprechend dem folgenden Reaktionsschema hergestellt.

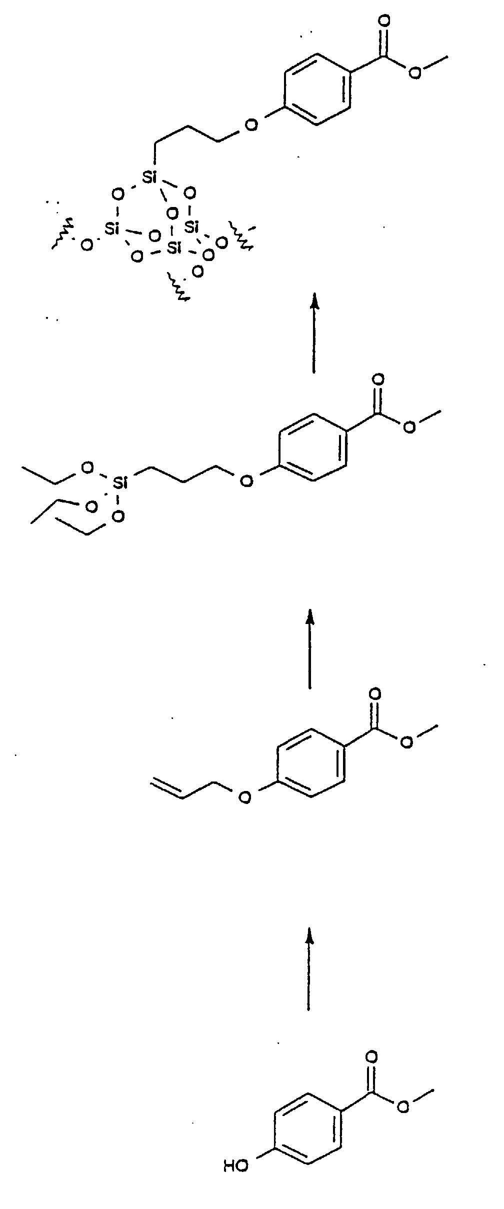

### Herstellung von 4-(3-Propen-1-yl-)oxy-benzoesäure-methvlester

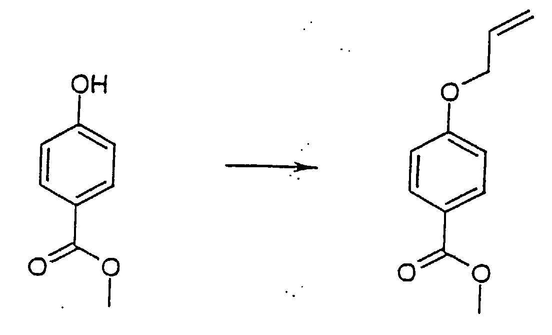

Analog zur Vorschrift von Beispiel 1 wurden 32 g Methylparaben (4-Hydroxy-benzoesäure-methylester) mit 35,2 g 3-Brom-1-propen und 54,5 g Natriummethylat (30%ige Lösung in Methanol) umgesetzt.

Nach Destillation im Vakuum wurden 37,6 g 4-(3-Propen-1-yl-)oxy-benzoesäuremethylester erhalten, dessen physikalische Daten mit den für die gewünschte Verbindung (CAS-No.: [35750-24-4]) publizierten identisch waren.

### Herstellung des silanisierten Zwischenproduktes

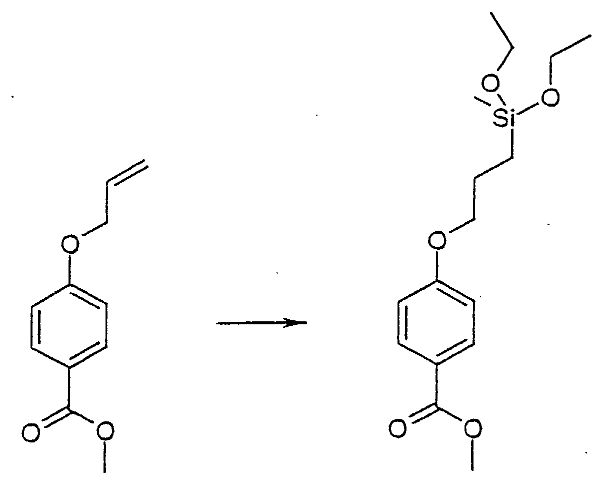

10,2 g 4-(3-Propen-1-yl-)oxy-benzoesäure-methylester wurden analog zur Vorschrift von Beispiel 2 in THF mit 14,8 g Methyldiethoxysilan umgesetzt. Es wurden 4 mg Hexachloroplatin(VI)säure-Hexahydrat als Katalysator eingesetzt.

Nach der beschriebenen Aufarbeitung wurden 17,7 g Rohprodukt erhalten.

### Kopplung des silanisierten Zwischenproduktes an Monospher® 25

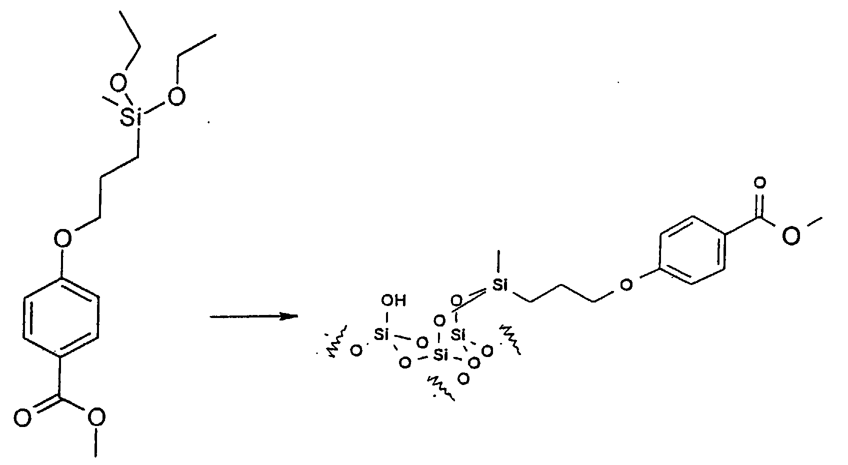

Analog zum im Beispiel 2 beschriebenen Ablauf wurde eine ethanolische Suspension von ca. 80 g Monospher® 25 hergestellt, die mit den 17,7 g Rohprodukt (silyliertes Methylparaben-Analogon) aus der vorhergehenden Synthese umgesetzt wurde. Die Dispersion wurde am Rotationsverdampfer auf einen Trockengehalt zwischen 30% und 40% Konjugat aufkonzentriert.

Zur Herstellung kosmetischer Dispersionen wurden Aliquote mit den berechneten Mengen der verschiedenen kosmetischen Öle versetzt (siehe Tabelle in Beispiel 2) und das Ethanol am Rotationsverdampfer abgezogen.

In den folgenden Beispielen für Haut- und Sonnenschutzformulierungen wurde das Eusolex® 9020/Monospher® 25-Konjugat von Beispiel 2 verwendet. Es ist jedoch auch möglich, das Eusolex® 9020/Monospher® 100-Konjugat von Beispiel 1 oder ein anderes erfindungsgemäßes Konjugat zu verwenden.

### Beispiel 5 Beispiele für Hautschutzformulierungen

### Hautschutzlotion (O/W)

| Rohstoff | INCI | Gew.% | |
|---|---|---|---|
| A Monospher Konjugat | | (1) | 5,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 6,00 |
| Miglyol 812 Neutralöl | Caprylic/Capric Triglyceride | (4) | 6,00 |
| | | | |

| B Propandiol-1,2 | Propylene Gylcol | (1) | 4,00 |
|---|---|---|---|
| Allantoin | Allantoin | (1) | 0,20 |
| Konservierungsmittel | | | q.s. |
| Wasser, demineralisiert | Aqua | | ad 100,00 |

### Herstellung:

Die Phase A wird auf 75°C und die Phase B auf 80°C erwärmt. Die Phase B wird unter Rühren langsam zu der Phase A gegeben. Das Gemisch wird homogenisiert und unter Rühren abgekühlt.

### Bemerkungen:

Viskosität 9200 mPas (Brookfield RVT, Sp. C, 10 Upm) bei 24°C
pH_{24°C}=5,0
Konservierungsmittel:
0,05% Propyl-4-hydroxybenzoat
0,15% Methyl-4-hydroxybenzoat

### Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg

### Hautschutzlotion (O/W)

| Rohstoff | INCI | Gew.% | |
|---|---|---|---|
| A Monospher Konjugat | | (1) | 1,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 3,00 |
| Imwitor 900 | Glyceryl Stearate | (4) | 3,00 |
| Lunacera M | Microwax | (6) | 1,00 |
| Luvitol EHO | Cetearyl Octanoate | (3) | 11,50 |
| Cetiol | Oleyl Oleate | (5) | 7,00 |
| Miglyol 812 Neutralöl | Caprylic/Capric Triglyceride | (4) | 7,00 |
| | | | |

| B Propandiol-1,2 | Propylene Gylcol | (1) | 4,00 |
|---|---|---|---|
| Allantoin | Allantoin | (1) | 0,20 |
| Konservierungsmittel | | | q.s. |
| Wasser, demineralisiert | Aqua | | ad 100,00 |

### Herstellung:

Die Phase A wird auf 75°C und die Phase B auf 80°C erwärmt. Die Phase B wird unter Rühren langsam zu der Phase A gegeben. Das Gemisch wird homogenisiert und unter Rühren abgekühlt.

### Bemerkungen:

Konservierungsmittel:
0,05% Propyl-4-hydroxybenzoat
0,15% Methyl-4-hydroxybenzoat

### Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg

### Beispiel 6 Beispiele für Sonnenschutzformulierungen

### Sonnenschutzlotion mit IR3535™ (O/W)

| Rohstoff | INCI | Gew.% | |
|---|---|---|---|
| A Monospher Konjugat | | (1) | 3,00 |
| Eusolex 6300 | 4-Methylbenzylidene Camphor | (1) | 3,00 |
| IR 3535™ | Ethyl Butylacetylaminopropionate | (1) | 10,00 |
| (-)-α-Bisabolol | Bisabolol | (1) | 0,30 |
| Montanov 68 | Cetearyl Alcohol (and) Cetearyl Glucoside | (2) | 4,00 |
| Myritol 312 | Caprylic/Capric Triglyceride | (3) | 2,00 |
| Mirasil CM 5 | Cyclomethicone | (4) | 2,00 |
| Mirasil DM 350 | Dimethicone | (4) | 1,00 |
| | | | |

| B Wasser, demineralisiert | Aqua | | ad 100 |
|---|---|---|---|
| Glycerin, 87%ig | Glycerin | (1) | 3,00 |
| Konservierungsmittel | | | q.s. |
| | | | |

| C Rhodicare S | Xanthan Gum | (4) | 0,50 |
|---|---|---|---|
| | | | |

### Herstellung:

Die Phasen A und B werden getrennt voneinander auf 75°C erwärmt. Die Phase C wird bei 75°C unter Rühren langsam zu der Phase B gegeben. Das Gemisch wird gerührt, bis es homogen ist. Anschließend wird die Phase A zu dem Gemisch gegeben. Das Gemisch wird gerührt, bis es homogen ist, und dann unter Rühren abgekühlt.

### Bemerkungen:

Konservierungsmittel:
0,05% Propyl-4-hydroxybenzoat
0,15% Methyl-4-hydroxybenzoat
0,30% Germall 115 (ISP, Frechen)

### Bezugsguellen:

(1) Merck KgaA, Darmstad
(2) Interorgana, Köln
(3) Henkel, KgaA, Düsseldorf
(4) Rhodia, Frankfurt

### Sonnenschutzmilch (O/W)

| Rohstoff | INCI | Gew.% | |
|---|---|---|---|
| A Monospher Konjugat | | (1) | 4,00 |
| Eusolex 6300 | 4-Methylbenzylidene Camphor | (1) | 4,00 |
| Emulgator E 2155 | Stearyl Alcohol (and) Steareth-7 (and) Steareth-10 | (2) | 3,00 |
| Teginacid H | Glyceryl Stearate (and) Cateth-20 | (2) | 2,00 |
| Luvitol EHO | Cetearyl Octanoate | (3) | 14,00 |
| Imwitor 900 | Glyceryl Stearate | (4) | 3,00 |
| Cetiol | Oleyl Oleate | (5) | 6,00 |
| Luncacera M | Mikrowax | (6) | 1,00 |
| Miglyol 812 Neutralöl | Caprylic/Capric Triglyceride | (4) | 4,00 |
| | | | |

| B Eusolex 232 | Phenylbenzimidazole Sulfonic Acid | (1) | 2,00 |
|---|---|---|---|
| Tris(hydroxymethyl)-aminomethan | Tromethamine | (1) | 1,07 |
| Propandiol-1,2 | Propylene Glycol | (1) | 4,00 |
| Allantoin | Allantoin | (1) | 0,20 |
| Konservierungsmittel | | | q.s. |
| Wasser, demineralisiert | Aqua | | ad 100,00 |
| | | | |

| C Carbopol ETD 2050 | Carbomer ETD 2050 | (7) | 0,25 |
|---|---|---|---|
| Wasser, demineralisiert | Aqua | | 30,00 |
| | | | |

| D Tris(hydroxymethyl)-aminomethan | Tromethamine | (1) | 0,25 |
|---|---|---|---|
| Wasser, demineralisiert | Aqua | | 4,00 |

### Herstellung:

Carbopol ETD 2050 wird in Wasser homogen dispergiert, um die Phase C zu erhalten. Die Phase D wird dann unter Homogenisieren in die Phase C eingetragen. Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Inhaltsstoffe der Phase B zugegeben, und die Phase B wird langsam unter Homogenisieren in die Phasen C/D eingetragen. Die Phase A wird unter Erhitzen gelöst und langsam unter Homogenisieren zugegeben.

### Bemerkungen:

Konservierungsmittel:
0,05% Propyl-4-hydroxybenzoat
0,15% Methyl-4-hydroxybenzoat

### Bezugsquellen:

(1) Merck KGaA, Darmstadt
(2) Th. Goldschmidt, Essen
(3) BASF, Ludwigshafen
(4) Hüls, Troisdorf AG, Witten
(5) Henkel, Düsseldorf
(6) H.B. Fuller GmbH, Lüneburg
(7) Goodrich, Neuss

### Sonnenschutzlotion (W/O)

| | Rohstoff | | Gew.% |
|---|---|---|---|
| A | Monospher Konjugat | (1) | 5,00 |
| | Eusolex HMS | (1) | 5,00 |
| | Eusolex OS | (1) | 5,00 |
| | Eusolex OCR | (1) | 5,00 |
| | Abil WE 09 | (2) | 5,00 |
| | Jojobaöl | (3) | 3,00 |
| | Cetiol V | (4) | 3,00 |
| | Prisorine 2021 | (5) | 2,00 |
| | Lunacera M | (6) | 1,80 |
| | Miglyol 812 Neutralöl | (7) | 3,00 |
| | | | |

| B | Glycerin (etwa 87%) | (1) | 2,00 |
|---|---|---|---|
| | Natriumchlorid | (1) | 0,40 |
| | Konservierungsmittel | (1) | q.s. |
| | Wasser, demineralisiert | | ad 100,00 |

### Herstellung:

Die Phase B wird auf 80°C und die Phase A auf 75°C erwärmt. Die Phase B wird langsam in die Phase A eingerührt. Das Gemisch wird homogenisiert und unter Rühren abgekühlt.

### Bemerkungen:

Konservierungsmittel:
0,05% Propyl-4-hydroxybenzoat
0,15% Methyl-4-hydroxybenzoat

### Bezugsguellen:

(1) Merck KGaA, Darmstadt
(2) Th. Goldschmidt AG, Essen
(3) H. Lamotte, Bremen
(4) Henkel KGaA, Düsseldorf
(5) Unichema, Emmerich
(6) H.B. Fuller, Lüneburg
(7) Hüls Troisdorf AG, Witten

### Sonnenschutzcreme (W/O)

| Rohstoff | INCI | Gew.% | |
|---|---|---|---|
| A Monospher Konjugat | | (1) | 2,50 |
| Eusolex T-2000 | mikron. Titandioxid | (1) | 2,50 |
| Eusolex 6300 | 4-Methylbenzylidene Camphor | (1) | 2,00 |
| Dehymuls E | Dicocoyl Pentyerythrityl Citrate (and) Sorbitan Sesquioleate (and) Cera Alba (and) Aluminium Stearate | (2) | 6.00 |
| Arlacel 989 | PEG-7 Hydrogenated Castor Oil | (3) | 1,00 |
| Bienenwachs | Cera Alba | (1) | 2,00 |
| Zinkstearat | Zinc Stearate | (1) | 2,00 |
| Cetiol J 600 | Oleyl Erucate | (2) | 6,00 |
| Cetiol V | Decyl Oleate | (2) | 6,00 |
| Cetiol OE | Dicaprylyl Ether | (2) | 5,00 |
| Dow Corning 200 (100 cs) | Dimethicone | (4) | 1,00 |
| DL-α-Tocopherolacetat | Tocopheryl Acetate | (1) | 1,00 |
| Vitamin-A-palmitat | Retinyl Palmitate | (5) | 0,50 |
| | | | |

| B Eusolex 232 | Phenylbenzimidazole Sulfonic Acid | (1) | 2,00 |
|---|---|---|---|
| Tris(hydroxymethy)-aminomethan | Tromethamine | (1) | 0,88 |
| Glycerin (etwa 87%) | Glycerin | (1) | 5,00 |
| Magnesiumsulfat Heptahydrat | Magnesium Sulfate | (1) | 1,00 |
| Allantoin | Allantoin | (1) | 0,20 |
| Konservierungsmittel | | | q.s. |
| Wasser, demineralisiert | | | ad 100,00 |

### Herstellung:

Zur Neutralisierung von Eusolex 232 wird das Tris(hydroxymethyl)-aminomethan im Wasser der Phase B gelöst, und Eusolex 232 wird unter Rühren zugegeben. Nach vollständiger Lösung werden die restlichen Rohstoffe der Phase B zugegeben und auf 80°C erwärmt. Die Phase A wird auf 75°C erwärmt. Die Phase B wird langsam in Phase A eingerührt, und das Gemisch wird unter Rühren abgekühlt.

### Bemerkungen:

Konservierungsmittel:
0,05% Propyl-4-hydroxybenzoat
0,15% Methyl-4-hydroxybenzoat

### Bezugsquellen:

(1) Merck KGaA, Darmstadt
(2) Henkel KGaA, Düsseldorf
(3) ICI, Essen
(4) Dow Corning, Düsseldorf
(5) Hoffmann La Roche, Schweiz

### Sonnenschutzgel (O/W)

### mit UV-A/B-Schutz

| | Rohstoff | | Gew.% |
|---|---|---|---|
| A | Monospher Konjugat | (1) | 2,00 |
| | Eusolex 2292 | (1) | 5,50 |
| | Oxynex K flüssig | (1) | 1,00 |
| | Luvitol EHO | (2) | 9,00 |
| | Dow Corning 200 (100 cs) | (3) | 2,00 |
| | Antaron V-220 | (4) | 2,00 |
| | Jojobaöl | (5) | 5,00 |
| | | | |

| B | Tris(hydroxymethyl)-aminomethan | (1) | 0,60 |
|---|---|---|---|
| | Konservierungsmittel | | q.s. |
| | Wasser, demineralisiert | | ad 100,00 |
| | | | |

| C | Pemulen TR-1 | (6) | 0,50 |
|---|---|---|---|
| | Wasser, demineralisiert | | 29,50 |
| | | | |

| D | Aloe Vera Gel 1:10 | (7) | 1,00 |
|---|---|---|---|
| | | | |

### Herstellung:

Das PemulenTR-1 wird im Wasser homogen dispergiert und vorgequollen, um die Phase C zu erhalten. Die Phase B wird unter Homogenisieren in die Phase C eingetragen. Die Phase A wird unter Erwärmen gelöst und langsam unter Homogenisieren zugegeben. Bei 35°C wird die Phase D zugesetzt und nochmals homogenisiert.

### Bemerkungen:

Konservierungsmittel:
0,05% Propyl-4-hydroxybenzoat
0,15% Methyl-4-hydroxybenzoat

### Bezugsquellen:

(1) E. Merck, Darmstadt
(2) BASF, Ludwigshafen
(3) Dow Corning, Düsseldorf
(4) GAF, Frechen
(5) Henry Lamotte, Bremen
(6) Goodrich, Neuss
(7) Galke, Gittelde

### Sonnenschutzspray (O/W)

| Rohstoff | INCI | Gew.% | |
|---|---|---|---|
| A Monospher Konjugat | | (1) | 3,00 |
| Eusolex 2292 | Octyl Methoxycinnamate | (1) | 7,50 |
| Eusolex HMS | Homosalate | (1) | 7,00 |
| Volpo S-2 | Steareth-2 | (2) | 0,40 |
| Volpo S-10 | Steareth-10 | (2) | 0,80 |
| Pemulen TR-2 | Acrylate/C 10-30 Alkyl Acrylate Crosspolymer | (3) | 0,18 |
| Hetester PHA | Propylene Glycol Isoceteth-3 Acetate | (4) | 5,00 |
| Performa V 825 | Synthetic Wax | (5) | 0,80 |
| Dow Corning 200 (100cs) | Dimethicone | (6) | 1,00 |
| Oxynex K flüssig | PEG-8 (and) Tocopherol (and) Ascorbyl Palmitate (and) Ascorbic Acid (and) Citric Acid | (1) | 0,10 |
| | | | |

| B Eusolex 232 | Phenylbenzimidazole Sulfonic Acid | (1) | 1,00 |
|---|---|---|---|
| Triethanolamin | Triethanolamine | (1) | ' 0,90 |
| Propandiol-1,2 | Propylene Glycol | (1) | 2,00 |
| Konservierungsmittel | | | q.s. |
| Wasser, demineralisiert | Aqua | | ad 100,00 |

### Herstellung:

Zur Neutralisierung von Eusolex 232 wird das Triethanolamin ins Wasser der Phase B gegeben, und Eusolex 232 wird unter Rühren zugegeben. Nach vollständiger Lösung werden die restlichen Rohstoffe der Phase B zugegeben und auf 80°C erwärmt. Die Phase A wird bis auf das Pemulen zusammengegeben und auf 80°C erwärmt. Dann wird das Pemulen in Phase A eingerührt. Die Phase B wird unter Rühren langsam zu der Phase A gegeben, und das Gemisch wird homogenisiert und unter Rühren abgekühlt.

### Bemerkungen:

Konservierungsmittel:
0,05% Propyl-4-hydroxybenzoat
0,15% Methyl-4-hydroxybenzoat

### Bezugsquellen:

(1) Merck KGaA, Darmstadt
(2) Croda, Nettetal
(3) Goodrich, Neuss
(4) ROVI, Schlüchtern
(5) New Phase, NJ 08554
(6) Dow Corning, Wiesbaden

## Patentansprüche

1. Konjugat, umfassend ein anorganisches Pigment und einen Wirkstoff auf der Basis organischer Verbindungen, der kovalent über eine Spacer-Gruppe an das anorganische Pigment gebunden ist, **dadurch gekennzeichnet, dass** die Spacer-Gruppe ein Siliciumatom oder ein Aluminiumatom enthält.

2. Konjugat nach Anspruch 1, **dadurch gekennzeichnet, dass** das anorganische Pigment eine Verbindung eines Metalls oder Halbmetalls ist.

3. Konjugat nach Anspruch 2, **dadurch gekennzeichnet, dass** die Verbindung eines Metalls oder Halbmetalls ein Oxid, Silikat, Phosphat, Carbonat, Sulfat oder Nitrid ist.

4. Konjugat nach Anspruch 3, **dadurch gekennzeichnet, dass** das Oxid Magnesiumoxid, Aluminiumoxid, Siliciumoxid, Zinkoxid, Ceroxid, Titanoxid, Zirconiumoxid, Manganoxid, Boroxid, Eisenoxid oder ein Gemisch dieser Oxide ist.

5. Konjugat nach Anspruch 3 oder 4, **dadurch gekennzeichnet, dass** das Oxid in Form von kugelförmigen monodispersen Oxidpartikeln vorliegt.

6. Konjugat nach Anspruch 3, **dadurch gekennzeichnet, dass** das Silikat ein Glimmer oder ein Talk ist.

7. Konjugat nach einem der Ansprüche 1 bis 6, **gekennzeichnet durch** eine Partikelgröße von 1 nm bis 250 µm.

8. Konjugat nach einem der Ansprüche 1 bis 7, **dadurch gekennzeichnet, dass** der Wirkstoff eine Verbindung ist, ausgewählt aus lichtabsorbierenden organischen Verbindungen, Substanzen mit antioxidativen und/oder radikalinhibierenden Eigenschaften, Repellentien, Konservierungsmitteln und Derivaten dieser Wirkstoffe.

9. Konjugat nach Anspruch 8, **dadurch gekennzeichnet, dass** die lichtabsorbierende organische Verbindung aus Derivaten von Aminobenzoesäure, Zimtsäure, Salicylsäure, Benzylidenkampfer, Phenylbenzimidazol, Diphenylacrylat, Triazin, Triazol, Benzophenon, Benzoylmethan, Diarylbutadienen und vinylgruppenhaltigen Amiden ausgewählt ist.

10. Konjugat nach Anspruch 9, **dadurch gekennzeichnet, dass** die lichtabsorbierende organische Verbindung photostabil ist.

11. Konjugat nach Anspruch 8, **dadurch gekennzeichnet, dass** die Substanzen mit antioxidativen und/oder radikalinhibierenden Eigenschaften aus Flavonoiden, Coumaranonen, Vitaminen und BHT ausgewählt sind.

12. Konjugat nach Anspruch 8, **dadurch gekennzeichnet, dass** die Repellentien aus Amiden und Derivaten davon ausgewählt sind.

13. Konjugat nach Anspruch 12, **dadurch gekennzeichnet, dass** die Repellentien aus N,N-Diethyl-3-methyl-benzamid, 3-[N-n-Butyl-N-acetyl]-aminopropionsäureethylester und N,N-Caprylsäurediethylamid ausgewählt sind.

14. Konjugat nach Anspruch 8, **dadurch gekennzeichnet, dass** die Konservierungsmittel aus Benzalkoniumchlorid, Benzoesäure und deren Salzen, Methylparaben, Ethylparaben, Propylparaben, Sorbinsäure und deren Salzen, Cetylpyridiniumchlorid, Cetrimoniumchlorid und Salicylsäure und deren Salzen ausgewählt sind.

15. Konjugat nach einem der Ansprüche 1 bis 14, **gekennzeichnet durch** die allgemeine Formel 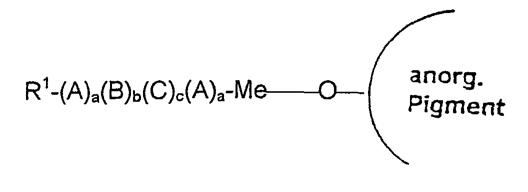 worin bedeuten:
R¹ die kovalent gebundene Wirkstoffgruppe,
A O, S oder NH,
B eine geradkettige oder verzweigte Alkylengruppe mit bis zu 20 Kohlenstoffatomen,
C eine geradkettige oder verzweigte Alkylenoxygruppe mit bis zu 20 Kohlenstoffatomen, wobei das Sauerstoffatom der Alkylenoxygruppe an die Gruppe B gebunden ist,
Me ein Siliciumatom oder ein Aluminiumatom,
a 0 oder 1,
b 0 oder 1,
c 0 oder 1,
wobei das Silicium- bzw. Aluminiumatom eine oder mehrere kovalente Bindungen zum anorganischen Pigment und/oder zur Wirkstoffgruppe ausbilden kann.

16. Konjugat nach Anspruch 15, **dadurch gekennzeichnet, dass** R¹ ausgewählt ist aus der Gruppe, bestehend aus 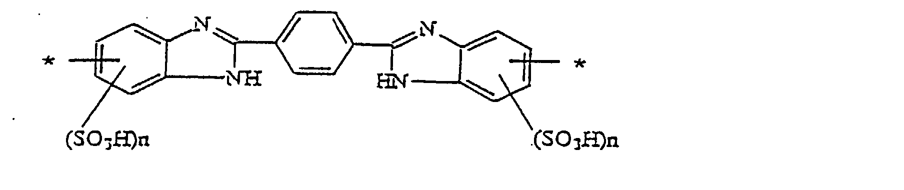 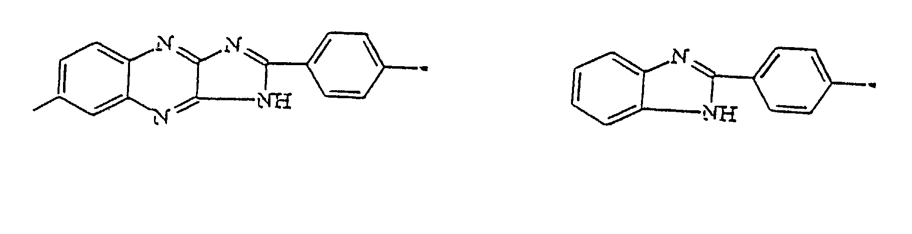 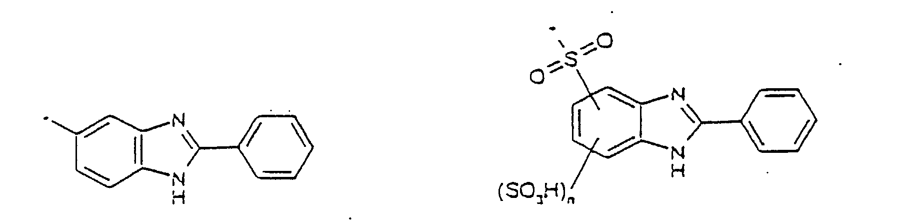 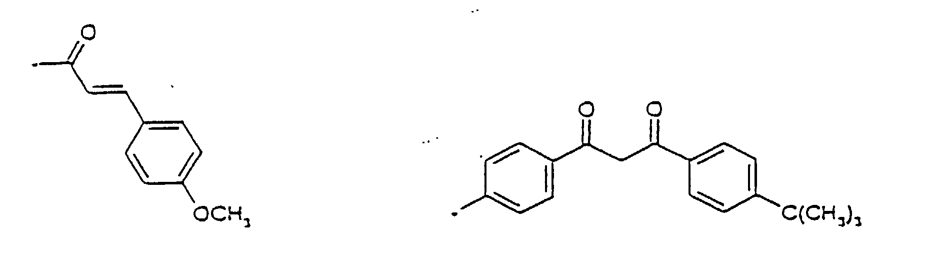 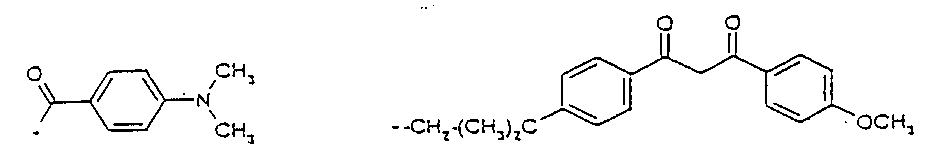 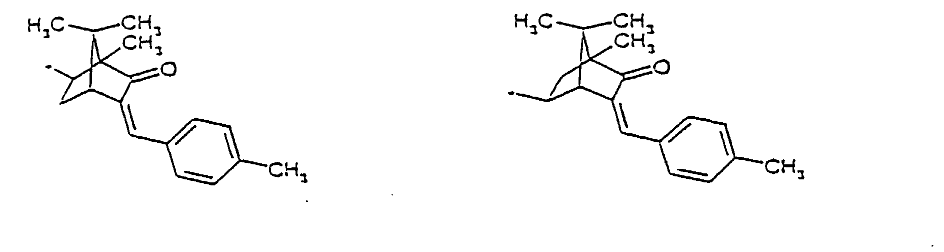 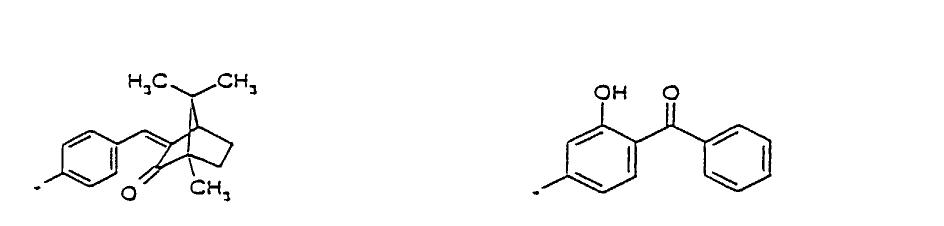 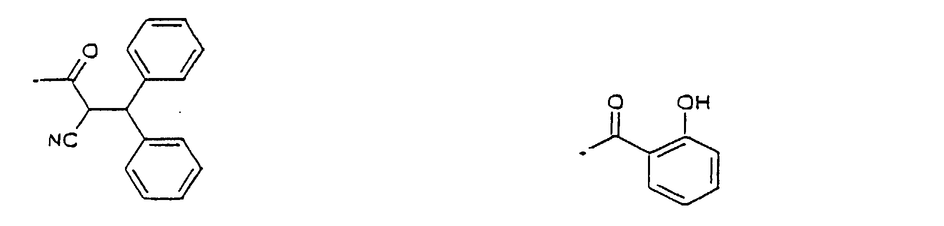 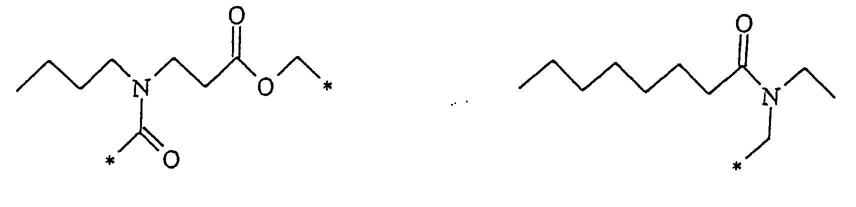 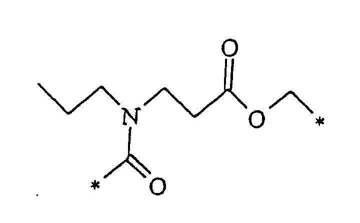 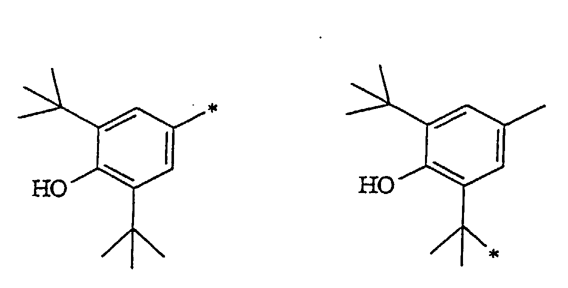 worin n 0, 1 oder 2 bedeutet, und * bedeutet die Bindung zur Spacer-Gruppe.

17. Konjugat nach einem der Ansprüche 1 bis 16, **dadurch gekennzeichnet, dass** der Wirkstoff wasserlöslich und/oder öllöslich ist.

18. Konjugat der Formel 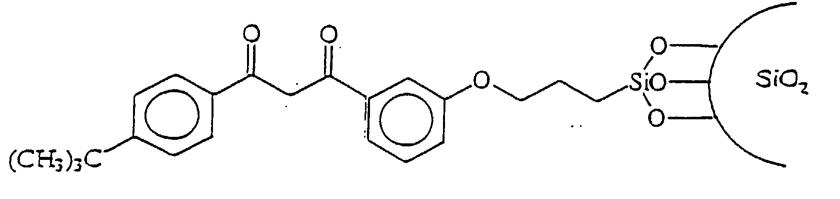 worin SiO₂ ein Pigment auf der Basis von Siliciumdioxid ist.

19. Konjugat der Formel 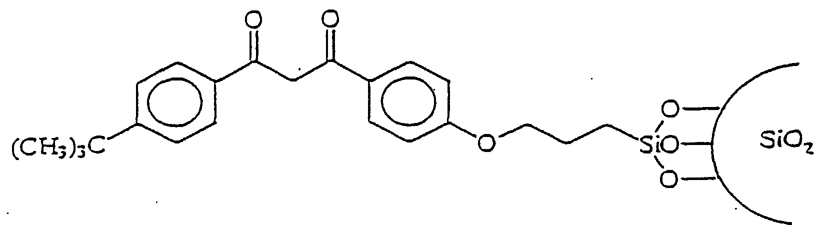 worin SiO₂ ein Pigment auf der Basis von Siliciumdioxid ist.

20. Konjugat der Formel 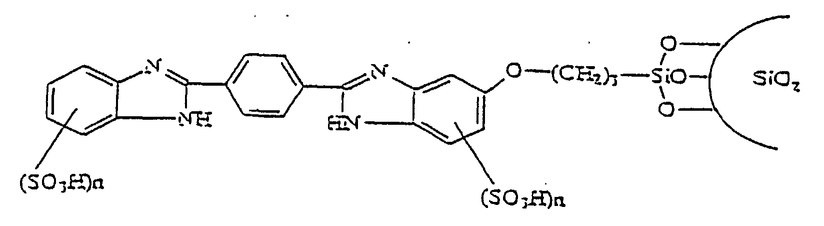 worin SiO₂ ein Pigment auf der Basis von Siliciumdioxid ist und n 0, 1 oder 2 bedeutet..

21. Konjugat der Formel 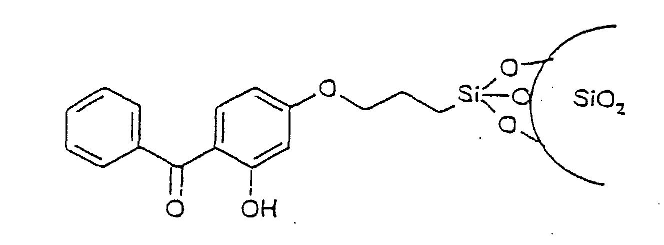 worin SiO₂ ein Pigment auf der Basis von Siliciumdioxid ist.

22. Konjugat der Formel 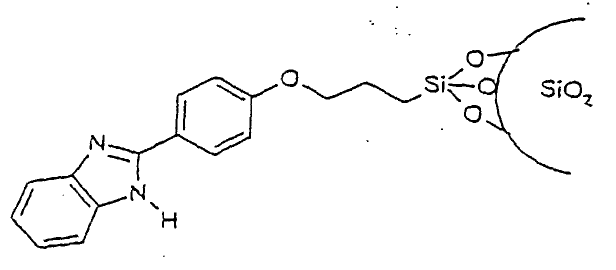 worin SiO₂ ein Pigment auf der Basis von Siliciumdioxid ist.

23. Konjugat der Formel 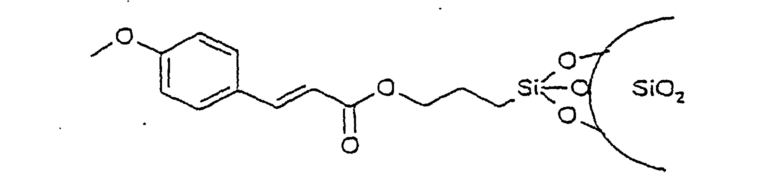 worin SiO₂ ein Pigment auf der Basis von Siliciumdioxid ist.

24. Konjugat der Formel 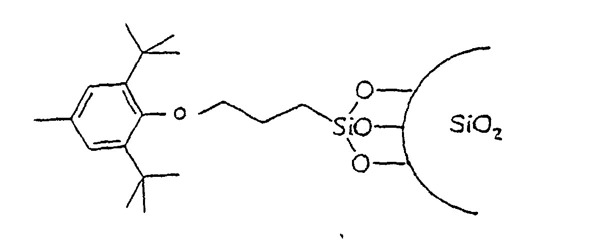 worin SiO₂ ein Pigment auf der Basis von Siliciumdioxid ist.

25. Konjugat der Formel 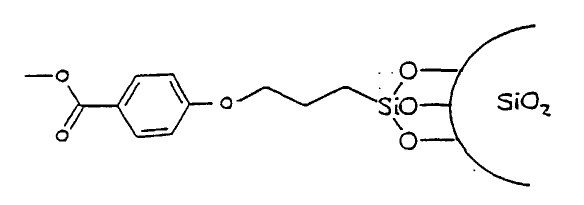 worin SiO₂ ein Pigment auf der Basis von Siliciumdioxid ist.

26. Dermatologische oder kosmetische Zusammensetzung, umfassend mindestens ein Konjugat nach einem der Ansprüche 1 bis 25 und mindestens einen kosmetisch, pharmazeutisch und/oder dermatologisch verträglichen Träger und/oder Hilfsstoff.

27. Dermatologische oder kosmetische Zusammensetzung nach Anspruch 26, **dadurch gekennzeichnet, dass** sie mindestens einen weiteren UV-Schutzstoff enthält.

28. Dermatologische oder kosmetische Zusammensetzung nach Anspruch 26 oder 27, **dadurch gekennzeichnet, dass** sie ein Antioxidationsmittel enthält.

29. Dispersion, umfassend ein Konjugat nach einem der Ansprüche 1 bis 25 und einen Ölbestandteil und/oder einen flüssigen Lichtfilter.

30. Verfahren zur Herstellung des Konjugats nach einem der Ansprüche 1 bis 25, umfassend
(a) das Umsetzen einer Verbindung, die mindestens zwei endständige reaktive Gruppen enthält, mit einem Wirkstoff auf der Basis organischer Verbindungen, um beide Verbindungen über eine der beiden reaktiven Gruppen kovalent aneinander zu binden, und
(b) das anschließende Umsetzen der in Schritt (a) erhaltenen Verbindung mit einem anorganischen Pigment, um die in Schritt (a) erhaltene Verbindung kovalent über die andere reaktive Gruppe an das anorganische Pigment zu bilden.

31. Verfahren zur Herstellung des Konjugats nach einem der Ansprüche 1 bis 25, umfassend
(a) das Umsetzen einer Verbindung, die mindestens zwei endständige reaktive Gruppen enthält, mit einem anorganischen Pigment, um die Verbindung und das Pigment über eine der beiden reaktiven Gruppen kovalent aneinander zu binden, und
(b) das anschließende Umsetzen der in Schritt (a) erhaltenen Verbindung mit einem Wirkstoff auf der Basis organischer Substanzen, um die in Schritt (a) erhaltene Verbindung kovalent über die andere reaktive Gruppe an den Wirkstoff zu bilden.

32. Verwendung des Konjugats nach einem der Ansprüche 1 bis 25 zur Herstellung einer Sonnenschutzformulierung oder einer Hautschutzformulierung.

33. Verwendung des Konjugats nach einem der Ansprüche 1 bis 25 zum Schutz der Hautzellen.

34. Verwendung nach Anspruch 33 zum Schutz der Langerhans-Zellen in der Haut.

35. Verwendung des Konjugats nach einem der Ansprüche 1 bis 25 zum Schutz der DNA.

36. Verwendung des Konjugats nach einem der Ansprüche 1 bis 25 zur Immunprotektion.
